(19) Europäisches Patentamt
European Patent Office
Office européen des brevets

(11) Numéro de publication: 0 577 915 A1

(12) # DEMANDE DE BREVET EUROPEEN

(21) Numéro de dépôt: **92870102.8**

(22) Date de dépôt: **09.07.92**

(83) Déclaration conformément à la règle 28(4) CBE(solution de l'expert)

(51) Int. Cl.5: **C12N 15/81**, C12N 1/18, A21D 8/04, C07K 15/00, C12N 9/12

Le demandeur a présenté une déclaration conformément à la règle 28 (4) CBE (remise d'un échantillon uniquement à un expert). Numéro(s) d'ordre de(s) dépôt(s): CBS 242.92

(43) Date de publication de la demande: **12.01.94 Bulletin 94/02**

(84) Etats contractants désignés: **BE**

(71) Demandeur: **N.V. ALGIST-BRUGGEMAN**
**Langerbruggekaai, 37**
**B-9000 Gent(BE)**

(72) Inventeur: **Thevelein, J.**
**Groeneweg, 46**
**B-3001 Leuven-Heverlee(BE)**
Inventeur: **Hohmann, S.**
**Koning Leopold III laan, 30**
**B-3001 Leuven-Heverlee(BE)**
Inventeur: **van Dijck, P.**
**J. Vandezandestraat, 21**
**B-3050 Oud-Heverlee(BE)**

(74) Mandataire: **Vanderperre, Robert et al**
**Bureau Vander Haeghen S.A.**
**Rue Colonel Bourg 108 A**
**B-1040 Bruxelles (BE)**

(54) **Souches de levures transformées de manière à posséder une résistance au stress et/ou un pouvoir fermentatif amélioré.**

(57) Des souches de levure transformées ou modifiées de manière à posséder une meilleure résistance au stress et/ou un métabolisme des sucres différents sont décrites. Elles possèdent par rapport à une souche non transformée, une modification d'au moins un de leurs systèmes généraux senseurs de glucose composés au moins :

- d'une protéine ayant une fonction de senseur général de glucose codée par le gène GGS1 ou un gène similaire ;
- d'une protéine transporteur membranaire (perméase) du glucose à faible affinité;
- d'un sucre kinase.

La modification d'au moins un des gènes GGS1 ou de ses allèles ou des gènes ayant, au moins partiellement, la même fonction que ce gène GGS1, et/ou de leur promoteur, et/ou de leur séquence 3'-flanquante, confère à la souche transformée des nouvelles propriétés pour sa production et/ou son utilisation comme levure à usage industriel.

EP 0 577 915 A1

## Domaine de l'invention

La présente invention est relative aux souches de levure transformées de manière à ce qu'elles possèdent une résistance au stress améliorée ou un métabolisme des sucres différent, comme par exemple un pouvoir fermentatif amélioré. La présente invention vise des transformations de souches de levure, ainsi que les outils (vecteurs) nécessaires à ces transformations. La présente invention, vise en tant que produit nouveau, les nouvelles souches de levure plus résistantes au stress et/ou ayant un métabolisme des sucres différent comme, par exemple,un pouvoir accru de transformation des sucres simples tels le glucose, obtenues par lesdites transformations, ainsi que les nouvelles levures de panification, de brasserie, de vinification et de manière générale toutes les biomasses composées de levures obtenues à partir desdites nouvelles souches.

## Description de l'art antérieur

Les levures de type Saccharomyces cerevisiae sont utilisées comme agent de fermentation des pâtes en boulangerie. Leur activité fermentative repose sur leur capacité à produire du dioxyde de carbone à partir des sucres présents dans la pâte ou ajoutés à celle-ci. Le pouvoir fermentatif est un critère de qualité important d'une levure de boulangerie.

La recherche fondamentale menée au cours de ces dernières années a confirmé que le tréhalose a la propriété de protéger les structures biologiques (protéines, membranes, organelles, cellules entières) des stress exercés sur les organismes. Une corrélation étroite existe entre une teneur élevée en tréhalose des levures de panification et, d'une part, leur bon niveau de résistance au stress, par exemple pendant le séchage, et, d'autre part, la préservation de leur pouvoir fermentatif au cours de la congélation.

A l'heure actuelle, les procédés de production de levure de boulangerie sont menés de façon à obtenir des levures dont la teneur en tréhalose soit suffisamment élevée (10 à 25% par rapport au poids sec), pour assurer une bonne stabilité des cellules à la conservation. Mais, d'une part, la recherche de levures riches en tréhalose n'est pas compatible avec l'obtention de levures très riches en protéines et, d'autre part, au moment où l'on utilise la levure, le tréhalose est rapidement mobilisé (catabolisé) et ce, dès le début de la fermentation de la pâte. Il en résulte une perte de résistance au stress importante des levures, qui constitue, par exemple, un inconvénient majeur si la pâte est stockée sous forme congelée car le stress de la congélation entraîne une importante perte du pouvoir fermentatif de la levure au sein des pâtons congelés. De manière générale, tout procédé ou toute manipulation entraînant une diminution de la vitesse de mobilisation du tréhalose de la levure pendant la fermentation confère un avantage commercial indéniable.

Sur un plan structurel, la teneur en tréhalose de la levure est dépendante de la tréhalose-6-phosphate-synthétase/phosphatase,l'enzyme responsable de la synthèse du tréhalose, et de l'activité de la tréhalase, l'enzyme responsable de l'hydrolyse du tréhalose. L'existence d'autres protéines affectant spécifiquement le métabolisme du tréhalose n'a pas encore été établie. Cependant, il est bien connu que l'activité de la protéine kinase AMP cyclique dépendante a une influence considérable sur la teneur en tréhalose de la levure, au moins et peut-être de manière non-exclusive, par son effet d'activation de la tréhalase. Une activité élevée de la protéine kinase provoque une activité élevée de la tréhalase et des teneurs en tréhalose faibles alors qu'une activité peu élevée de cette même enzyme se traduit par une activité plus faible en tréhalase et une teneur plus élevée en tréhalose.

Le mécanisme provoquant la mobilisation du tréhalose pendant la fermentation est complexe. Les sucres fermentescibles induisent la mobilisation du tréhalose apparemment en provoquant l'activation de la tréhalase et l'inactivation de la tréhalose-6-phosphate-synthétase/phosphatase, par l'intermédiaire de signaux régulateurs. Toutefois, la mobilisation du tréhalose provoquée par les sucres fermentescibles seuls n'est qu'un phénomène transitoire. En absence de sources d'azote, les cellules commencent à utiliser le sucre présent dans le milieu pour synthétiser à nouveau du tréhalose. En présence de sources d'azote, le tréhalose est dégradé de manière complète et permanente. Ceci est dû à la fois à la stimulation induite par la source d'azote de l'activation de la tréhalase et de l'inactivation de la tréhalose-6-phosphate-synthétase/phosphatase. Il est par ailleurs connu que l'effet de la source d'azote requiert la présence de sucres fermentescibles dans le milieu de culture. Le mécanisme exact de l'effet complexe selon lequel la fermentation induit la mobilisation du tréhalose n'est toujours pas éclairci, notamment en ce qui concerne la nature des signaux de régulation, spécifiques ou non.

En général, le rôle principal de la levure dans des procédés de fermentation est de produire du dioxyde de carbone et de l'éthanol. Comme mentionné ci-dessus, la production de dioxyde de carbone par la levure de boulangerie provoque la levée de la pâte, tandis que les levures de brasserie, de vinification et autres produisent de l'éthanol dans le moût vinique, brassicole ou les autres substrats fermentescibles. Dans tous les cas, une levure ayant une capacité de fermentation plus élevée réduit le temps nécessaire à la réalisation de la fermentation et/ou permet la conduite de celle-ci avec moins de levure. L'éthanol et le dioxyde de carbone sont formés par les levures à partir de glucides par la voie de la glycolyse. Aujourd'hui, la recherche de l'étape limitante de la conversion du glucose en éthanol et dioxyde de carbone par les levures est un sujet controversé. La surexpression des gènes codant pour les enzymes glycolytiques dont on pense qu'elles sont ou pourraient être des étapes limitantes de la glycolyse, n'augmente pas la vitesse de fermentation. Apparemment, l'étape limitante de la glycolyse se situe à un autre niveau et notamment au niveau des systèmes de transport membranaire. Ceci est en accord avec certaines observations montrant qu'en situation de carence d'azote, le transport transmembranaire du glucose et le flux glycolytique décroissent simultanément.

Des cellules de Saccharomyces cerevisiae se développant sur des sources de carbone non fermentescibles, comme le glycérol ou l'éthanol, réagissent à l'addition de glucose ou de sucres aisément fermentescibles avec une remarquable diversité d'effets régulateurs (Thevelein JM 1988, Exp. Mycol., 12, pp. 1-12 ; Thevelein JM 1991, Mol. Microbiol., 5, pp. 1301-1307). Dans un premier temps, il apparait des effets rapides post-traductionnels comprenant :

- la stimulation de la voie RAS-adénylate-cyclase, du turnover du phosphatidylinositol, de la synthèse du fructose-2,6-bisphosphate ;
- la stimulation de l'entrée et de la sortie du calcium, accompagnée d'une augmentation transitoire nette du niveau intracellulaire de $Ca^{2+}$ ;
- l'activation de la tréhalase ;
- l'activation de l'$H^+$-ATPase membranaire ;
- l'inactivation de la fructose-1,6-bisphosphatase (Fbase), de la malate déshydrogénase, de l'isocitrate lyase, de la phosphoénolpyruvate carboxykinase ;
- l'inactivation du transport du glucose par le système à haute affinité, du transport du galactose et du maltose.

Dans un second temps, des effets plus tardifs, exercés au niveau de la transcription des gènes, sont induits comprenant :

- l'effet de répression bien connu du glucose sur la respiration des mitochondries, sur la gluconéogenèse et sur plusieurs autres systèmes comme ceux du transport et du catabolisme des glucides métabolisés plus lentement ;
- la répression par le glucose des gènes *CTT1*, *UBI4*, *SSA3* ou *HSP12* ;
- l'induction par le glucose de divers gènes glycolytiques comme le gène *PDC* codant pour la pyruvate décarboxylase ;
- l'induction par le glucose de la synthèse des ARN ribosomaux et de la synthèse des protéines.

Jusqu'à ce jour, pour induire tous ces effets régulateurs, la seule étape nécessaire connue, probablement commune à tous les effets induits par le glucose, est la phosphorylation du glucose.

Par ailleurs, il a été démontré que le mutant *fdp1* de la levure (déficient dans sa capacité à désactiver la fructose diphosphatase) est également déficient dans l'activation induite par le glucose de la voie RAS-adénylate cyclase. Ce mutant est incapable de croître sur des sucres fermentescibles (glucose, mannose, saccharose) mais il n'est déficient dans aucune des enzymes de la glycolyse. L'addition de sucres rapidement fermentescibles à des cellules du mutant *fdp1*, cultivées préalablement sur des sources de carbone non fermentescibles, provoque une diminution rapide de l'ATP et une hyperaccumulation intracellulaire de sucres phosphorylés (Van de Poll KW, Kerkenaar A & Schamhart DHJ 1974, J. Bacteriol., 117, pp. 965-970 ; Van de Poll KW & Schamhart DHJ 1977, Mol. Gen. Genet., 154, pp. 61-66).

Récemment un gène supresseur rétablissant la croissance du mutant *fdp1* sur sucres fermentescibles a été isolé et nommé *FPS1*. Ce gène ne rétablit néanmoins pas les déficiences de régulation du mutant *fdp1*, ni au niveau des signaux AMP cyclique induits par le glucose, ni au niveau de l'inactivation de la fructose-1,6-diphosphatase. Le supresseur *FPS1* ne rétablit pas la teneur très faible en tréhalose du mutant *fdp1* - (Van Aelst L, Hohmann S, Zimmermann K, Jans AWH & Thevelein JM 1991, EMBO J., 10, pp. 2095-2104).

**Brève description de l'invention**

La présente invention décrit un procédé d'obtention de souches de levure ayant, sous toutes conditions, au moins l'une des propriétés suivantes :

- un plus haut niveau de base en tréhalose ;
- une vitesse de mobilisation réduite du tréhalose ;
- une résistance au stress plus importante ;

- un métabolisme différent des sucres et notamment une capacité d'utilisation des sucres plus élevée ;

et les souches obtenues par ce procédé.

Ce procédé consiste à transformer au moins un des systèmes généraux senseurs de glucose desdites souches et notamment un de leurs gènes *GGS1* ou tout autre gène similaire.

Au sens de l'invention, le système appelé senseur général de glucose dans les levures est un système composé d'au moins :

- une protéine transporteur membranaire (perméase) du glucose ;
- une sucre-kinase ;
- une protéine ayant une fonction de senseur général de glucose, codée par le gène *GGS1* ou tout autre gène ayant une fonction similaire, le gène *GGS1* étant ci-après précisément décrit.

Cette dernière protéine a deux principales fonctions :

- d'une part la régulation de l'entrée du glucose dans les cellules de levure ;
- d'autre part l'activation d'un certain nombre de signaux conduisant aux changements métaboliques induits par la présence de glucose, dont notamment les signaux commandant la synthèse du tréhalose, sa mobilisation ou sa dégradation ; des signaux commandant probablement d'autres mécanismes de réponse au stress et des signaux agissant sur le catabolisme d'autres glucides comme le fructose, le galactose, le maltose ou le saccharose.

En effet, il a été vérifié que le produit du gène *GGS1* de Saccharomyces cerevisiae joue un rôle crucial dans le contrôle du métabolisme du carbone dans la levure. Sa délétion entraîne une réduction du niveau de tréhalose, une réduction simultanée de la résistance au stress et une augmentation de l'entrée du glucose dans les cellules de levure. L'expression du gène *GGS1* est réprimée pendant la fermentation par l'action combinée de la source de sucres fermentescibles et de la source d'azote présentes dans le milieu de culture. Par ailleurs son expression semble augmentée sous l'action de stress comme l'augmentation de la température.

La présente invention concerne une souche de levure transformée de manière à posséder une résistance au stress et/ou un métabolisme des sucres différent, comme notamment un pouvoir fermentatif amélioré, caractérisée par le fait d'une part, que cette souche possède par rapport à une souche non transformée (dite de type sauvage) une modification d'au moins un de ses systèmes généraux senseurs de glucose composé au moins :

- d'une protéine ayant une fonction de senseur général de glucose codée par le gène *GGS1* ou un gène similaire;
- d'une protéine transporteur membranaire (perméase) du glucose à faible affinité ;
- d'une sucre kinase.

et, d'autre part, par le fait que la modification du ou des gènes codant pour ces protéines et/ou de leur promoteur et/ou de leur séquence 3'-flanquante confère à la souche transformée de nouvelles propriétés pour sa production et/ou son utilisation comme levure à usage industriel.

Suivant une particularité de l'invention, la souche de levure transformée possède, par rapport à la souche non transformée, une modification d'au moins un de ses gènes *GGS1* ou de ses allèles ou des gènes ayant au moins partiellement la même fonction que ce ou ces gènes *GGS1* ou une modification de leur promoteur ou de leur séquence 3'-flanquante.

Dans une forme de réalisation particulière, la souche de levure transformée résulte du fait qu'un ou plusieurs gènes *GGS1* ou ses allèles ont été mis sous promoteur constitutif rendant son expression au moins partiellement indépendante de toute régulation par le glucose et/ou par l'azote présents dans le milieu de culture ou tout autre promoteur, comme les promoteurs forts, inductibles ou conditionnels.

Suivant un développement de l'invention, la souche de levure transformée est caractérisée en ce qu'au moins un gène *GGS1*, son promoteur ou sa séquence 3'-flanquante est muté, de manière à ce que sa mutation entraîne spécifiquement une activation de la synthèse du tréhalose et/ou une inactivation au moins partielle de la mobilisation du tréhalose. Par exemple, des mutations du gène *GGS1* peuvent être conduites dans ce but par mutagenèse dirigée, soit en supprimant par délétion les sites de phosphorylation reconnus par les systèmes de régulation dépendant de l'AMP cyclique, de la protéine codée par *GGS1*, soit par des manipulations génétiques spécifiques de la région 3' du gène *GGS1*, afin de prévenir la dégradation de l'ARN messager induite par les nutriments.

La mutation du gène *GGS1* est de préférence identique à celle de son allèle *ggs1*−3.

possible, par sa surexpression, la formation d'une teneur plus élevée en tréhalose, sans effets secondaires sur le métabolisme des sucres, par exemple sur la vitesse de fermentation.

La souche de levure transformée est notamment plus résistante au stress que la souche non transformée, soit qu'elle acquiert dans les mêmes conditions de culture une plus grande résistance au stress, soit que la perte de sa résistance au stress est retardée, soit qu'elle cumule ces deux propriétés. Sa résistance au stress est indépendante ou moins dépendante du glucose et/ou de l'azote présents dans son milieu de culture ou de fermentation.

La résistance des levures au stress est définie comme la résistance des levures aux phénomènes tels que l'augmentation de pression osmotique, l'augmentation de température, le séchage ou la congélation, phénomènes qui altèrent la viabilité des cellules et leur vitesse de transformation des sucres comme le glucose, le fructose, le saccharose, le maltose ou le galactose. Une amélioration de la résistance au stress se traduit pour les cellules de levure (et les biomasses de levures) par au moins l'une des propriétés suivantes :

- meilleure résistance au séchage ;
- meilleure résistance à la pression osmotique, notamment dans les pâtes sucrées ;
- meilleure résistance à la congélation ;
- meilleure survie dans les pâtes boulangères congelées.

Ces propriétés pouvant se trouver combinées entre elles.

Dans une autre forme de réalisation de l'invention, l'un au moins des complexes généraux senseurs de glucose est modifié de manière à permettre une entrée plus rapide du glucose mais à une vitesse telle que la glycolyse est assurée. Cette entrée plus rapide du glucose peut être, par exemple, obtenue par délétion du gène *GGS1* ou d'un autre gène similaire. L'équilibre entre la vitesse d'entrée du glucose et la glycolyse peut être assurée, par exemple, en surexprimant ou en renforçant par augmentation du nombre de copies, les gènes de la glycolyse ou les gènes codants pour des transporteurs de phosphate, ou tout autre gène intervenant dans le métabolisme des glucides, dans les souches de levures dont au moins un complexe général senseur de glucose a été transformé dans le but d'accroître la glycolyse. Le gène *FPS1* dont il est intéressant de renforcer l'expression dans ce cadre, semble coder pour un transporteur de phosphate. Selon une autre alternative, des transformations du gène *GGS1* ou de tout autre composante du complexe senseur général de glucose, comme des changement de promoteurs, des mutations ponctuelles peuvent être introduites de sorte que l'effet limitant de l'entrée du glucose soit moins important, provoquant de ce fait (ou en combinaison avec une ou plusieurs des manipulations décrites ci-dessus) une augmentation du métabolisme des sucres, notamment de la fermentation. Les souches ainsi transformées présentent par rapport à des souches non transformées une augmentation de leur activité fermentative caractérisée par la vitesse de transformation des sucres présents en éthanol et dioxyde de carbone et/ou un rendement de croissance amélioré.

Est également inclus dans la présente invention, toute modification du senseur général de glucose et notamment du gène *GGS1* afin de modifier les contrôles exercés sur le système RAS-adénylate cyclase, le turnover du phosphatidylinositol, l'$H^+$-ATPase, l'inactivation protéolytique de la fructose-1,6-bisphosphatase (Fbase), la voie principale de la répression glucose, l'entrée de $K^+$, l'induction de la pyruvate décarboxylase, les systèmes de régulation induits par l'azote, les signaux de régulation commandant les métabolismes des sucres. On obtient ainsi des souches dont la transformation du système général senseur de glucose est dirigée de manière à obtenir un changement spécifique des signaux de régulation induits par la présence de glucose et/ou des signaux de régulation commandant le métabolisme des sucres.

Est également inclus dans la présente invention l'utilisation du gène *GGS1* ou de l'un de ses allèles placé sous le contrôle d'un promoteur constitutif comme marqueur de sélection dominante positif pour la transformation des levures (par exemple levures industrielles) basée sur l'augmentation de la résistance au stress provoquée par leur surexpression. Le test de résistance au stress peut consister à faire subir à une culture de cellules un choc thermique de 50°C pendant 10 minutes au bain-marie, puis à refroidir rapidement la culture sur glace et enfin à effectuer un dénombrement des survivants.

L'invention vise des méthodes pour obtenir les souches désirées par des moyens classiques, c'est-à-dire sans avoir recours aux recombinaisons génétiques par ADN transformant.

La souche de levure transformée est avantageusement une souche de Saccharomyces cerevisiae. Elle est de préférence une souche de levure de panification.

L'invention concerne également l'utilisation de souches de levure transformées pour la production d'alcool et la production de boissons, de mélanges nutritionnels, de protéines hétérologues et de biomasse de levures.

Les souches de levure de panification obtenues et les levures de panification obtenues avec ces levures possèdent l'une des caractéristiques suivantes par rapport à la souche non transformée cultivée dans les mêmes conditions :

- meilleure résistance au séchage;
- meilleure résistance aux chocs osmotiques, notamment dans les pâtes sucrées;
- meilleure résistance à la congélation;

- meilleure survie dans les pâtes boulangères congelées ou de préférence plusieurs de ces caractéristiques.

Les souches de levure obtenues présentent un métabolisme différent des sucres et en particulier une transformation des sucres plus rapide, ce qui est important économiquement. Si cette transformation des sucres est une multiplication des levures avec apport d'oxygène, plus la biomasse nécessaire comme levure de panification, de brasserie, de distillerie, de vinification, comme levure-aliment ou diététique, pour la production de protéines hétérologues, sera produite dans un temps court. Si cette transformation des sucres par les levures est une fermentation produisant de l'éthanol et du dioxyde de carbone, c'est-à-dire par exemple une fermentation panaire, brassicole, vinique ou cidricole, plus cette fermentation sera courte. Par ailleurs, il peut être intéressant de modifier le métabolisme d'un sucre donné dans un but donné, par exemple pour commander certaines synthèses.

L'invention vise enfin plus particulièrement des nouvelles souches de levure de panification et notamment des nouvelles souches de levure fraîches et sèches de panification. Elle vise notamment les levures fraîches très actives, c'est-à-dire à haut pouvoir fermentatif, contenant au moins 55% de protéines brutes (N x 6,25) et au moins 10% de tréhalose, c'est-à-dire des levures sèches conservant bien et pouvant être séchées sans perte de force fermentative importante. L'invention vise également les nouvelles levures sèches correspondantes. L'invention vise de nouvelles levures de panification résistantes à la congélation et plus particulièrement des souches et des levures de panification gardant un niveau de tréhalose suffisant, même après un premier départ en fermentation lors de l'incorporation des levures dans les pâtes congelées. La résistance au séchage, par exemple pour l'obtention de levures sèches très actives, et la résistance à la congélation sont des propriétés importantes pour les levures de panification, mais aussi pour les levures de vinification, pour les levures de brasserie. Toutes ces levures d'ensemencement ont et auront de plus en plus besoin d'être conservées et transportées et d'être soumises à des stress comme, par exemple, le séchage ou la congélation. Dans leurs conditions d'utilisation, elles peuvent être soumises à un choc osmotique, par exemple en panification, lorsque les levures sont incorporées dans des pâtes sucrées. L'espèce de levure en cause dans toutes ces fermentations est le plus souvent Saccharomyces cerevisiae.

Ces particularités et détails de l'invention ainsi que d'autres ressortiront de la description détaillée des formes de réalisation particulières et des exemples décrits ci-dessous en faisant référence aux dessins ou figures ci-joints, dans lesquels :

- la figure 1 est la séquence nucléotidique du gène *GGS1* comprenant le promoteur, la partie codante et la séquence 3' flanquante. La séquence déduite d'acides aminés du produit d'expression du gène *GGS1* est également indiquée.
- la figure 2 donne un shéma de construction de la délétion de *GGS1* par recombinaison homologue. Un fragment SalI-BglII du gène *GGS1* est délété in-vitro puis remplacé par le marqueur *LEU2*. La construction résultante a été échangée dans le génome avec le gène originel de type sauvage *GGS1*, après digestion par SalI et BglII et transformation par le fragment d'ADN linéaire d'une souche de levure *leu2*. Les enzymes de restriction sont ainsi désignées : B : BamHI; Bg : BglII; E : EcoRI; H : HindIII; S : SalI; Sa : Sau3A; Sc : SacI; Sp : SphI.
- la figure 3 est un modèle illustrant le fonctionnement du complexe général senseur de glucose d'une levure. Dans les cellules de type sauvage, l'entrée du glucose est contrôlée et assurée par un complexe formé (au moins) d'un transporteur de glucose à faible affinité, d'une sucre kinase, et du produit d'expression du gène *GGS1*. Ce complexe a deux fonctions principales. La première consiste à restreindre et à réguler l'entrée du glucose. La seconde consiste à activer toute une série de voies de signalisation et probablement toutes les voies de signalisation induites par le glucose. Dans les mutants *ggs1* (*ggs1−1=fdp1* ; *ggs1−3=byp1*) le complexe est totalement ou partiellement non fonctionnel. L'entrée du glucose n'est plus limitée, ce qui provoque une hyperaccumulation intracellulaire rapide de sucres phosphorylés avec une diminution concomitante du niveau d'ATP. L'activation des voies de signalisation par le glucose est perdue. La restauration de la croissance par le gène supresseur *FPS1* est probablement due à la reconstitution du pool de phosphate intracellulaire par transport de phosphate extracellulaire.
- la figure 4 illustre l'expression de *GGS1* pendant la croissance diauxique de la souche M5 de type sauvage sur milieu glucose-éthanol (recherche des ARN messagers par la technique d'hybridation d'ARN sur membrane dite Northern Blot). Bande 1 : ARN messagers extraits après 7 heures de culture (cellules en phase exponentielle de croissance sur milieu contenant du glucose); bande 2 : Après 11 heures (cellules en phase de transition, après épuisement du glucose) et bande 3 : Après 14 heures (cellules en phase exponentielle de croissance sur l'éthanol produit à partir du glucose). La bande *URA3* sert de témoin d'hybridation.

- la figure 5 illustre l'expression de *GGS1* (recherche des ARN messagers par Northern Blot) dans des cellules en phase exponentielle de croissance (souche sauvage M5) dans un milieu de culture contenant du glycérol (bande 1) ou du glucose (bande 2). La bande *URA3* sert de témoin d'hybridation.
- la figure 6 illustre l'expression du gène *GGS1* (recherche des ARN messagers par Northern Blot) dans la souche de type sauvage M5, en situation de carence d'azote puis après réaddition d'une source d'azote dans le milieu de culture. Bande 1: ARN messagers de cellules en phase exponentielle de croissance sur milieu de culture complet contenant du glucose ; Bande 2 : Après 30 minutes de carence en azote ; Bande 3 : Après 24 heures de carence en azote ; Bande 4 : 10 minutes après réaddition d'asparagine 10 mM ; Bande 5 : 60 minutes après réaddition de d'asparagine 10 mM. Remarque : l'abbréviation mM signifie millimole/litre.
- les figures 7a et 7b illustrent la cartographie des vecteurs réplicatifs ou intégratifs portant le gène *GGS1* sous le contrôle des promoteurs forts des gènes *PDC1* ou *PGK* : (a) Vecteurs contenant le gène marqueur d'auxotrophie *LEU2* ; (b) Vecteurs contenant le gène marqueur d'auxotrophie *URA3*. Les positions relatives des divers sites de restriction sont indiquées sur les vecteurs ( par exemple EcoRI, BamHI, NarI, KpnI...).
- la figure 8 montre un diagramme dont la courbe décrit la variation de la teneur en tréhalose en fonction du temps dans trois souches de levures transformées : Souche M5 contenant YEplac195 (●), YEp195PDC1/GGS1 (**o**) ou YIp211PGK/GGS1 (▲).

**Description détaillée**

Le mutant de levure *ggs1−1*
des sources de carbone fermentescibles et n'accumule pas de tréhalose lorsqu'il est cultivé sur des sources de carbone non fermentescibles. La croissance sur des sources de carbone fermentescibles peut être rétablie par l'introduction d'un plasmide portant un gène supresseur appelé *FPS1*, sans rétablir les niveaux normaux de tréhalose dans le mutant. Un autre allèle de *GGS1*, *ggs1−3*
*byp1−3*)a été isolé dans des souches inaptes à croître sur glucose et privées de l'un des deux gènes codant pour les sous-unités catalytiques de la phosphofructokinase (Breitenbach-Schmitt I, Schmitt HD, Heinisch J & Zimmermann FK, 1984, Mol. Gen. Genetics, 195, pp. 536-540). La mutation *ggs1−3*
provoque une phase de latence importante lors de la culture sur des sources de carbone fermentescibles, sans affecter sensiblement le niveau de tréhalose (Tableau 1A).

Le criblage d'une librairie génomique de levure construite dans le plasmide multicopie YEp13 (Broach JR, Strathern JN & Hicks JB 1979, Gene, 8, pp. 121-133) pour la sélection des clones qui complémentent la mutation *ggs1−1*
(Sengstag C & Hinnen A 1987, Nucleic Acids Research, 15, pp. 233-246) pour la sélection des clones qui complémentent la mutation *ggs1−3*,
Ce gène rétablit la croissance sur des sources de carbone fermentescibles ainsi que les défauts de régulation des deux mutants *ggs1−1*et *ggs1−3*,lorsqu'il est cloné avec l'un ou l'autre des vecteurs monocopie ou multicopie.

Le gène *GGS1* a été séquencé complètement sur les deux brins. La séquence complète (promoteur, partie codante et séquence 3' flanquante) est montrée dans la figure 1. Une comparaison avec les séquences d'ADN présentes dans les bases de séquences de gène de l'EMBL (European Molecular Biology Laboratory, Postfach 10.2209 D-6900 Heidelberg) et GENBANK (Bolt Beranek and Newman Inc., 10 Moulton street, Cambridge MA 02238, USA) a montré que ce gène avait déjà été séquencé antérieurement sur un fragment contenant le gène *VMA2* codant pour une sous-unité de l'$H^+$-ATPase vacuolaire (Nelson H, Mandiyan S & Nelson N 1989, J. Biol. Chem., 264, pp. 1775-1778). Cependant les nombreuses différences constatées entre la séquence publiée et la séquence établie dans la figure 1, explique pourquoi ces auteurs n'ont pas détecté la partie codante du gène *GGS1*.

La présente séquence est en majeure partie identique à celle du gène *CIF1* (Gonzalez MI, Stucka R, Blazquez MA, Feldmann H & Gancedo C 1992, Yeast, 8, pp. 183-192). Le mutant ***cif1*** est un autre allèle de *GGS1* possédant un phénotype de révertant partiel de ***ggs1−1***.En effet, les mutants ***cif1*** croissent sur glucose mais pas sur fructose (Navon G, Shulman RG, Yamane T, Eccleshall TR, Lam K-B, Baronofsky JJ & Marmur J 1979, Biochemistry, 18, pp. 4487-4499).

La séquence de la protéine codée par *GGS1* (déduite de la séquence nucléotidique du gène) ne présente aucune homologie avec d'autres protéines de fonction connue. Elle contient deux sites possibles de phosphorylation par la protéine kinase AMP cyclique dépendante, en particulier aux positions 270 (KKES*) et 411 (KKGS*). Ceci semble indiquer que le produit du gène *GGS1* est aussi contrôlé et inactivé par phosphorylation.

Les allèles *ggs1−1*

F & Fink GR 1983, Methods in Enzymology, 101, pp. 211-218). Dans ce but, le plasmide YIplac211 (Gietz RD & Sugino A 1988, Gene, 74, 527-534) a été utilisé en y intégrant le gène *GGS1* porté par un fragment HindIII-BamHI de 4,6 kb. Il contient un seul site de restriction SphI dans sa séquence 3'-flanquante.

Le plasmide a été linéarisé par l'enzyme de restriction SphI et transféré dans la souche *ggs1−1* MV6807 (Van Aelst L, Hohmann S, Zimmermann FK, Jans AWH & Thevelein JM 1991, EMBO J., 10, pp. 2095-2104) et la souche *ggs1−3*

Zimmermann FK 1992, J. Bacteriol., 174, pp. 4183-4188). L'intégration monocopie a été contrôlée par la technique d'hybridation d'ADN sur membrane dite Southern Blot. L'ADN chromosomique du transformant a alors été digéré par l'enzyme HindIII afin de libérer le plasmide intégré en même temps que l'allèle mutant. Après ligation, l'ADN a servi à la transformation d'Escherichia coli. Les plasmides résultants contenaient donc le fragment HindIII-BamHI de 4,6 kb avec les allèles des mutants *ggs1−1*

ont été complètement séquencés sur les deux brins. L'allèle *ggs1−1*

en position aa 64 au lieu d'un résidu glycine dans l'allèle de type sauvage (*GGS1*), tandis que le l'allèle *ggs1−3*

de type sauvage.

Des parties du gène *GGS1* ont été délétées dans le génome de deux souches de levure haploïdes de laboratoire, SP1 (Toda T, Uno I, Ishikawa T, Powers S, Kataoka T, Broek D, Cameron S, Broach J, Matsumoto K & Wigler M 1985, Cell, 40, pp. 27-36) et une souche diploïde, M5 (Hohmann S & Zimmermann FK 1986, Curr. Genet., 11, pp. 217-225), en appliquant la méthode décrite par Rothstein (1983, Methods in Enzymology, 101, pp.202-211).

Un fragment BamHI-HindIII de 4,6 kb a été sous-cloné dans le vecteur YIplac211 (figure 2). Le plasmide YIp211-GGS1B/H qui en résulte a été digéré par les enzymes de restriction SalI et BglII pour déléter 150 paires de bases dans la partie codante de *GGS1* (positions 874-1024) et la portion délétée a été remplacée par un fragment de 2,6 kb SalI-BglII dérivé du vecteur YEp13 qui contient le gène *LEU2* de la levure. Cette construction a été digérée par les enzymes de restriction BamHI et HindIII pour libérer l'insert contenant l'allèle *ggs1Δ::LEU2*, intégré par transformation dans les souches SP1 ou M5 qui sont déficientes en leucine et permettre le remplacement de la copie génomique (ou de l'une des deux copies de la souche diploïde M5) de *GGS1* par recombinaison homologue. La délétion de *GGS1* dans le génome a été contrôlée et confirmée par Southern Blot sur les transformants prototrophes pour la leucine. Les souches obtenues après délétion de *GGS1* dans la souche SP1 ont été appelées SP1−*ggs1Δ*

sporulation de la souche diploïde M5, dans laquelle une copie du gène *GGS1* a été délétée, ont été appelées YSH6.5.-2A (descendant ayant le gène de type sauvage *GGS1*) et YSH6.5.-2D (descendant ayant le gène *ggs1Δ*).

Les transformants *ggs1Δ* ont un phénotype qui ressemble à celui du mutant *ggs1−1*

pas sur des sucres fermentescibles de carbone (par exemple glucose, fructose, mannose et saccharose) mais croissent par contre sur des milieu de culture contenant du glycérol, du galactose et/ou du maltose. Ils présentent une teneur fortement réduite en tréhalose (Tableau 1A).

Tous les résultats du tableau 1 ont été obtenus avec des souches cultivées sur YPglycérol (2% de bactopeptone, 1% d'extraits de levure et 3% de glycérol) et récoltées en phase stationnaire. La teneur en tréhalose a été mesurée par la méthode de Neves (Neves MJ, Jorge JA, François JM & Terenzi HF 1991, FEBS letters, 283, pp. 19-22), en utilisant la tréhalase d'Humicola et le test glucose-oxydase/peroxydase.

**TABLEAU 1** : Teneur en tréhalose des souches *GGS1*, *ggs1-1*, *ggs1-3* et *ggs1*Δ.

| Souches | Tréhalose en micromoles/g poids humide |
|---|---|
| Tableau 1A. | |
| SP1 | 4,30 |
| SP1-*ggs1*Δ | 0,50 |
| YSH6.5.-2A (*GGS1*) | 17,10 |
| YSH6.5.-2D (*ggs1*Δ) | 1,70 |
| YSH3.131.-6A (*ggs1-3*) | 6,50 |
| Tableau 1B. | |
| YSH6.5.-1C (*ggs1*Δ) | 2,00 |
| YSH6.5.-1C/YlpGGS1 | 41,50 |
| YSH6.5.-1C/Ylpggs1-1 | 6,00 |
| YSH6.5.-1C/Ylpggs1-3 | 24,90 |
| Tableau 1C. | |
| S15-5B (*tpk1*Δ *tpk2*$^{w1}$ *tpk3*Δ) | 15,40 |
| S15-5B-*ggs1*Δ (*tpk1*Δ *tpk2*$^{w1}$ *tpk3*Δ *ggs1*Δ) | 0,10 |
| S22-5D (*tpk1*Δ *tpk2*Δ *tpk3*$^{w1}$) | 13,90 |
| S22-5D-*ggs1*Δ (*tpk1*Δ *tpk2*Δ *tpk3*$^{w1}$ *ggs1*Δ) | 0.05 |

L'ensemble de ces résultats indiquent que le produit du gène *GGS1* possède une fonction cruciale tant pour le contrôle de l'entrée du glucose dans la cellule que pour assurer à la levure une teneur appropriée en tréhalose. Le fait que le mutant *ggs1*Δ soit tout comme le mutant *ggs1−1*
important de fonctions de régulations induites par le glucose, montre l'existence d'un système général senseur de glucose. Le senseur général de glucose a notamment une fonction de détection du glucose, codée par le gène *GGS1*. Le système général senseur de glucose comporte au moins une protéine de transport membranaire (perméase) du glucose à faible affinité, une sucre kinase et le produit du gène *GGS1* comme illustré dans la figure 3. Le gène *GGS1* a une double fonction :

1) réguler l'entrée du glucose dans les cellules de levure.

2) activer un certain nombre de voies de signalisation conduisant aux différentes régulations induites par le glucose. Dans les mutants *ggs1*, le contrôle de l'entrée du glucose et de l'activation des voies de régulation est déficient.

Afin de comparer l'effet des allèles mutants et de l'allèle sauvage de *GGS1* dans le cadre d'un même patrimoine génétique, c'est-à-dire la même souche hôte de départ, les plasmides YIplac211 contenant *ggs1−1*,
de M5 : YSH6.5.-1C, ce qui a abouti aux souches YSH6.5.-1C/YIpGGS1, YSH6.5.-1C/YIpggs1-1 et YSH6.5.-1C/YIpggs1-3. Les souches résultantes se comportent essentiellement de la même manière que les mutants originaux. La réintroduction du gène *GGS1* rétablit le niveau de tréhalose tandis que le clonage de l'allèle *ggs1−3*le rétablit en partie et que le clonage de l'allèle *ggs1−1*
mesure (tableau 1B).

Pour examiner si le faible niveau de tréhalose dans les mutants *ggs1*Δ est une conséquence de la suractivation du système RAS adénylate cyclase/protéine kinase AMP cyclique dépendante ou une conséquence plus directe de l'absence du produit du gène *GGS1*, on a introduit la délétion *ggs1*Δ de la même manière que celle décrite précédemment, dans deux souches présentant une activité protéine kinase AMP cyclique dépendante fortement réduite. Les deux souches utilisées ont été S15-5B (génotype : *Mata his3 leu2 ura3 trp1 ade8 tpk1::URA3 tpk2$^{w1}$ tpk3::TRP1*) et S22-5D (génotype : *Mata his3 leu2 trp1 ade8 tpk1::URA3 tpk2::HIS3 tpk3$^{w1}$*). Ces deux souches présentent des délétions en deux des trois gènes *TPK* codant pour la sous-unité catalytique de la protéine kinase AMP cyclique dépendante et une mutation ponctuelle partiellement inactivante dans le troisième gène (Nikawa J, Cameron S, Toda T, Ferguson KW & Wigler M 1987, Gene and Development, 1, 931-937).

Dans ces deux souches, la délétion du gène *GGS1* mène à un niveau de tréhalose très bas (tableau 1C). Ceci indique le besoin spécifique en produit du gène *GGS1* qu'exige la synthèse du tréhalose plutôt qu'un effet indirect issu de la voie de l'AMP cyclique. Des résultats récents montrent que dans les mutants de levure thermosensibles pour la synthèse de l'AMP cyclique, les sources d'azote ou d'autres nutriments comme les phosphates ou les sulfates, sont toujours capables d'induire l'activation de la tréhalase dans des conditions non permissives. Ces observations confirment l'existence d'un mécanisme autre que l'AMP cyclique impliqué dans l'induction de la mobilisation du tréhalose.

L'expression du produit du gène *GGS1* a été étudiée pendant le processus de fermentation dans deux souches non transformées différentes, la souche haploïde SP1 et la souche diploïde M5. Dans les deux souches, l'expression du gène *GGS1* est faible pendant la fermentation, en particulier quand du glucose est présent. Lorsque le glucose est épuisé, l'expression du gène augmente de manière significative et continue à progresser pendant plusieurs heures. Les résultats obtenus avec la souche M5 sont illustrés par la figure 4 : Bande 1 : ARN messagers extraits après 7 heures de culture (cellules en phase exponentielle de croissance sur milieu contenant du glucose); bande 2 : Après 11 heures (cellules en phase de transition, après épuisement du glucose) et bande 3 : Après 14 heures (cellules en phase exponentielle de croissance sur l'éthanol produit à partir du glucose). Dans cette figure et la suivante la bande *URA3* sert de témoin d'hybridation. De manière similaire, lorsque les hybridations par Northern Blot sont effectuées pour estimer l'expression de GGS1 dans des cellules en phase exponentielle de croissance de la souche de type sauvage M5 cultivée sur milieu soit glucose, soit glycérol, le niveau d'expression de *GGS1* (figure 5) est bien plus élevé dans les cultures effectuées sur glycérol (bande 1) que dans les cultures effectuées sur glucose (bande 2).

De plus, l'effet de l'azote sur l'expression de *GGS1* a pu être étudié en présence de sucres fermentescibles. La carence d'azote provoque une augmentation rapide (en moins de 30 minutes) et importante du niveau d'ARN messagers de *GGS1*. Une nouvelle addition d'azote provoque une diminution très rapide (en moins de 10 minutes) du niveau d'ARN messagers de *GGS1*. Ceci est illustré par la figure 6 avec : Bande 1: ARN messagers de cellules (souche M5) en phase exponentielles de croissance sur milieu de culture complet contenant du glucose ; Bande 2 : Après 30 minutes de carence en azote ; Bande 3 : Après 24 heures de carence en azote ; Bande 4 : 10 minutes après réaddition d'asparagine 10 mM ; Bande 5 : 60 minutes après réaddition d'asparagine 10 mM.

Ainsi il apparaît une très bonne corrélation entre la formation du tréhalose et l'expression de *GGS1* d'une part, et entre la mobilisation du tréhalose et la répression de *GGS1* d'autre part. Le gène *GGS1* apparait être régulé de la même façon que les gènes *CTT1*, *UBI4*, *HSP12*, *ADH2* et *SSA3*, qui eux aussi sont soumis à un contrôle combiné par le glucose et l'azote. Le contrôle du glucose est différent de la voie principale de répression glucose des cellules de levure, qui n'est pas affectée par la carence en azote.

Il est intéressant de noter que le groupe de gènes *CTT1* est déréprimé par une activité basse et réprimé par une activité haute du système RAS adénylate cyclase/protéine kinase, conditions connues respectivement pour augmenter ou pour réduire la teneur en tréhalose. En outre, on sait qu'un choc thermique peut induire l'expression de *HSP12*, *CTT1* et *UBI4* et provoquer une augmentation de la teneur en tréhalose. L'analyse de l'influence de la température sur le niveau d'expression de *GGS1* a été entreprise. Pour ce faire, le gène *LacZ* (codant pour l'enzyme betagalactosidase) a été mis sous le contrôle du promoteur de *GGS1*, dans un vecteur multicopie (de type YEp) selon une technique classique (Guarente L & Ptashne M 1981, Proc. Natl. Acad. Sci. USA, 78, pp. 2199-2203 ; Rose M, Casadaban MJ & Botstein D 1981, Proc. Natl. Acad. Sci. USA, 78, pp. 2460-2464). Le vecteur ainsi obtenu a été cloné dans une souche de type sauvage. Les souches ainsi transformée ont été mises en incubation à différentes températures (23 °C, 34 °C, 37 °C, 40 °C et 42 °C) pendant 30-70 minutes, puis l'activité betagalactosidasique mesurée. Cette expérience a montré que dans les conditions d'un choc thermique et notamment à 37-40 °C, le promoteur de *GGS1* induit une augmentation significative du niveau d'expression. Le gène *GGS1* semble donc se comporter comme un gène de type Heat Shock.

En général, la corrélation entre la teneur en tréhalose et l'expression de *GGS1* apparaît très forte. Le niveau d'expression faible de *GGS1* pendant la fermentation dans un milieu complet (glucose + azote) est responsable au moins partiellement du faible niveau de tréhalose dans ces conditions. En outre, la mobilisation rapide du tréhalose dès le début de la fermentation des levures est due à la disparition rapide de l'ARN messager de *GGS1*.

L'expression du gène *GGS1* à partir d'un promoteur constitutif augmente l'expression du gène *GGS1* pendant la fermentation et empêche la disparition rapide de son ARN messager pendant la phase d'initiation de la fermentation. Ceci entraîne un niveau de tréhalose plus élevé et, par conséquent, également un degré de résistance au stress plus élevé pendant la fermentation et retarde la disparition rapide du tréhalose et la perte de résistance au stress concomitante pendant la phase d'initiation de la fermentation. De plus son niveau d'expression n'est plus dépendant de la température.

Par ailleurs, comme alternative au changement de promoteur, la mutagenèse dirigée sur des sites particuliers du gène *GGS1*, par exemple afin de supprimer les sites de phosphorylation reconnus par la protéine kinase AMP cyclique dépendante, peut être employée pour obtenir l'effet souhaité. Il en est de même de la manipulation génétique spécifique de la région 3' aval du gène afin d'empêcher la dégradation du messager induite par les nutriments.

Pour éviter les effets secondaires de la surexpression du gène *GGS1* sur d'autres parties du métabolisme, comme la vitesse de fermentation, l'allèle ***ggs1−3***ou d'autres allèles aboutissant à des propriétés phénotypiques similaires, peuvent être utilises. L'allèle ***ggs1−3***
(tableaux 1A et 1B) tout en étant déficient dans les fonctions de contrôle de régulations exercés par *GGS1*, par exemple, l'inhibition feed-back du transport du glucose. L'allèle ***ggs1−3***présent dans le fragment BamHI-HindIII de 4,8 kb intégré au vecteur YIplac211 (= plasmide YIplac211-ggs1-3) et cloné dans la souche DH5-alpha d'Escherichia coli, a été déposé le 4 mai 1992, au Centraalbureau voor schimmelcultures, Baarn, Pays-Bas, sous le numéro d'accès CBS 242.92.

**Exemple 1**

Ce premier exemple décrit le clonage de la région codante de *GGS1* sous le contrôle des promoteurs forts ou constitutifs *PDC1* et *PGK*. Le gène *GGS1* et les promoteurs *PDC1* et *PGK* ont été amplifiés par PCR en utilisant l'ADN génomique provenant de la souche diploïde de levure M5 de type sauvage, selon une technique classique (Saiki RK, in PCR methods, a guide to methods and applications., Academic Press Inc., San Diego, 1990, pp. 13-20). Les oligonucléotides d'amorce 5' et 3' (primer 5' et primer 3') employés pour la méthode PCR sont indiqués ci-dessous :

1. Pour *PDC1* : primer 5' -998 à -976
5' GAAGGCCAAATCAAGGCGGGAAG 3'
2. Pour *PDC1* : primer 3' -14 à -34

5' GCGGGATCCACTGTGTTATTTTGCGTGAG 3'
BamHI

Références 1 + 2 : Butler & Connell 1988, Curr. Gen., 14, pp.405-412
3. Pour *PGK* : primer 5' -542 à -34

5' GTCGGTACCGTGGCCTCTTATCGAGAA 3'
KpnI

4. Pour PGK : primer 3' -31 à -50

5' GTCGGATCCATTACTTCCTTGATGATCTG 3'
BamHI

Références 3 + 4 : Ogden et al 1986, Mol. Cell. Biol., 6, pp. 4335-4343
5. Pour *GGS1* : primer 5'

5' CTGGGATCCATAGAACTATGACTACGG 3'
BamHI

6. Pour *GGS1* : primer 3'

5' GAC<u>TCTAGA</u>GTTATGCGGTGTGAACAGCG 3'
**XbaI**

Remarque : il existe un site de restriction KpnI dans le promoteur de *PDC1* en aval du site de fixation du primer 5'

Taille des fragments amplifiés :

- Promoteur *PDC1* : 881 pb (-14 à -894)
- Promoteur *PGK* : 512 pb (-31 à -542)

Gène *GGS1* : 1839 pb (-1 à -8 + 1488 pb de partie codante + 343 pb de séquences 3' non traduites).

Les réactions PCR sont effectuées de la manière suivante : Dans un microtube à centrifuger de 500 microlitres, sont ajoutés 10 microlitres de tampon pour Taq polymérase (100 mM Tris pH 8,4, 15 mM $MgCl_2$, 500 mM KCl, 0,1% W/V gélatine); 16 microlitres d'un mélange des quatre dNTPs à 1,25 mM chacun ; les deux oligonucléotides d'amorce ou primers à une concentration finale de 1 micromole par litre chacun ; 1 microgramme d'ADN génomique total ; 2,5 U de Taq polymérase et de l'eau jusqu'à un volume final de 100 microlitres.

Le tout est recouvert avec 50 microlitres d'huile minérale. Le premier cycle comprend 7 minutes à 94 °C, 90 secondes à 51 °C et 2 minutes à 72 °C. Après ce cycle, on effectue 38 autres cycles comprenant 1 minute à 94 °C, 1 minute à 51 °C et 90 secondes à 72 °C. Dans le cycle final, les échantillons sont incubés pendant 3 autres minutes à 72 °C afin d'obtenir un ADN complet comprenant 2 brins. Les produits de PCR sont chargés sur un gel agarose à bas point de fusion et séparés par électrophorèse. Les bandes intéressantes sont isolées et purifiées par le kit de purification de l'ADN "Magic PCR Preps®" de Promega (2800 Woods Hollow Road, Madison, WI 53711-5399, USA). Les fragments d'ADN purifiés sont ensuite ligués et digérés par les enzymes de restriction appropriées.

La région codant *GGS1* est ensuite clonée sous le contrôle des promoteurs *PDC1* et *PGK*. Les plasmides YEplac195, YEplac181, YIplac211 et YIplac128 (Gietz RD & Sugino A 1988, Gene, 74, 527-534) ont été digérés par les 2 enzymes de restriction BamHI et XbaI. Les fragments de vecteurs linéarisés ont été purifiés par électrophorèse sur gel. Le fragment *GGS1* amplifié par PCR a été digéré par les mêmes enzymes et intégré dans les mêmes vecteurs. Dans une seconde étape, les vecteurs contenant le gène *GGS1* ont été digérés par les enzymes de restriction KpnI et BamHI et les vecteurs ont été à nouveau épurés par électrophorèse sur gel. Les fragments *PDC1* ou *PGK* amplifiés par PCR ont été ensuite clonés dans les vecteurs.

En appliquant ces méthodes, les vecteurs suivants ont été obtenus pour la surexpression du gène *GGS1* : YIp128PDC1/GGS1, YIp128PGK/GGS1, YEp181PDC1/GGS1, YEp181PGK/GGS1 (cartographies en figure 7a) et YEp195PDC1/GGS1, YEp195PGK/GGS1, YIp211PDC1/ggs1, YIp211PGK/GGS1 (cartographies en figure 7b). Ces vecteurs ont servi à la transformation de la souche diploïde M5. Les vecteurs du type YIp, contenant *LEU2* ou *URA3*, ont préalablement été linéarisés par EcoRV, qui coupe les gènes *LEU2* ou *URA3* au niveau d'un site unique, puis intégrés par recombinaison homologue dans la copie génomique de respectivement *leu2* et *ura3*. Les transformants ont été sélectionnés pour leur prototrophie à la leucine ou à l'uracile et testés à la fois par PCR et Southern Blot. La préparation et la manipulation des acides nucléiques, ainsi que la transformation de la levure ou d'<u>Escherichia coli</u> ont été réalisés en utilisant des méthodes classiques (Sambrook KJ, Fritsch EF & Maniatis T, 1989, Molecular cloning : A laboratory manual 2nd ed., Cold Spring Harbor Laboratory Press, CSH, New-York ; Sherman F, Fink GR & Hicks JB 1986, Methods in yeast genetics, Cold Spring Harbor Laboratory Press, CSH, New-York).

Par la suite, la mobilisation du tréhalose induite par le glucose a été mesurée dans les transformants. Pour ce faire, les transformants ont fait l'objet d'une culture sur YPG (2% bactopeptone, 1 % d'extraits de levure et 3% de glycérol) dans le cas de vecteurs intégratifs (type YIp) ou SDglycérol-uracile (Sherman F et al 1986, ibid.) dans le cas de vecteurs épisomaux (type YEp). Les cellules ont été récoltées à DO600nm d'environ 0,5 et remises en suspension dans un milieu de culture frais (YPG ou SDglycérol-uracile) à 30 °C. 20 minutes après la remise en suspension des cellules, du glucose est ajouté à la culture pour avoir une concentration finale de 100 mM et des échantillons sont prélevés pour déterminer la teneur en tréhalose à des intervalles de temps différents.

La figure 8 est un diagramme dont la courbe décrit la variation en fonction du temps de la teneur en tréhalose de 3 souches transformées contenant ou non le gène *GGS1* sous le contrôle d'un promoteur constitutif. Les souches suivantes ont été testées : La souche M5 contenant le plasmide multicopie YEp195PDC1/GGS1 (○), la souche M5 contenant le plasmide intégratif monocopie YIp211PGK/GGS1 (▲) et comme témoin la souche M5 contenant le vecteur YEplac195 (●).

Ces résultats montrent que la mobilisation du tréhalose est fortement réprimée dans les souches contenant le gène *GGS1* sous le contrôle d'un promoteur fort ou constitutif. Ces souches de levure transformée sont donc plus résistantes aux stress car leur teneur en tréhalose est devenue partiellement indépendante de la présence de glucose et/ou d'azote.

De manière surprenante, il a été constaté que, dans les cellules transformées portant le gène *GGS1* sous promoteur *PDC1* ou *PGK*, la Vmax (Vitesse maximale) caractérisant la cinétique d'entrée du glucose dans les cellules, est doublée. Ceci devrait conduire à une augmentation de la vitesse de fermentation desdites souches.

**Exemple 2**

Dans un second exemple l'allèle *ggs1−3*
constitutifs dans les mêmes vecteurs utilisés dans l'exemple 1. Pour ce faire, l'allèle *ggs1−3*
PCR en utilisant l'ADN du plasmide YIplac211-ggs1-3 (dépôt CBS n° 242-92). L'allèle *ggs1−3*
introduit de la même manière que précédemment décrit pour le gène *GGS1*, dans le même jeu de vecteurs.

Pour éviter le recours au génie génétique dans la construction des souches, les souches de levures industrielles ou leur descendants haploïdes peuvent être mutagénisés par des techniques classiques. Après la mutagénisation, les gènes *GGS1* avec leur propre promoteur sont isolés et clonés par PCR, en utilisant l'ADN génomique issu de clones provenant d'une cellule unique isolée parmi les cellules mutagénisées, et en introduisant , à chaque fois, le gène *GGS1* obtenu dans une souche *ggs1*Δ. Les transformants qui en résultent peuvent ensuite être sélectionnés sur le critère de mobilisation du tréhalose induite par les nutriments ou sur la perte de résistance au stress induite par les nutriments. Si une mutation supprimant la mobilisation du tréhalose induite par les nutriments ou la perte de résistance au stress induite par les nutriments est trouvée, elle peut être introduite dans le patrimoine génétique de souches industrielles par croisements successifs entre haploïdes descendants de souches industrielles de levures et les souches haploïdes mutagénisées originelles où une mutation intéressante de *GGS1* ou de son promoteur a été trouvée. Le devenir de la mutation dans la descendance peut être suivi par PCR si le phénotype recherché ne s'exprime pas clairement dans les premières générations de croisement. Le nombre de clones à tester au départ peut être éventuellement réduit par une première sélection rapide des mutants originels montrant une résistance accrue au stress. Le test de résistance au stress consiste à faire subir à une culture de cellules un choc thermique de 50°C pendant 10 minutes au bain-marie, puis à refroidir rapidement la culture sur glace et enfin à effectuer un dénombrement des survivants.

Le même principe est applicable aux souches contenant l'allèle *ggs1−3*.L'allèle *ggs1−3*peut être introduit par des croisements successifs dans le génotype de souches industrielles ou leur descendants haploïdes.

**Exemple 3**

La délétion ou la manipulation spécifique du gène *GGS1* ou d'un ou plusieurs autres composants du complexe général senseur de glucose supprime l'étape limitante principale de la fermentation. La surexpression de gènes dans ces nouveaux mutants de délétion augmente la vitesse de l'ensemble du processus général de fermentation des levures.

A titre d'exemple, le produit du gène *FPS1* rétablit la vitesse de fermentation initiale, mesurée grâce à la production d'éthanol, dans le mutant *ggs1−1*

TABLEAU 2

| Formation d'éthanol dans des souches *ggs1−1* | | |
|---|---|---|
| Souches | Formation d'éthanol (micromole/DO600nm) | |
| | 30 min. | 60 min. |
| LVA1531 (*ggs1−1*)<br>LVA4012(*ggs1−1* | 0,85 | 2,60 |

Pour obtenir les résultats du tableau 2, la souche *ggs1−1*
milieu YPD (2% bactopeptone, 1% d'extraits de levure et 2% de glucose), puis diluée dans du milieu YPD frais et enfin cultivée à nouveau jusqu'à l'obtention d'une suspension de cellules de DO600nm = 0,5. Les cellules ont alors été récoltées par centrifugation, lavées avec du milieu YPD frais et resuspendues dans du milieu YPD frais. Pour la souche *ggs1−1*,

de galactose) a été utilisé sauf au niveau de la remise en suspension finale qui a été également faite dans du milieu YPD. La production d'éthanol a été mesurée dans les cultures remises en suspension en prélevant des parties aliquotes à des intervalles de temps donnés, en sédimentant les cellules et en déterminant la teneur en éthanol au moyen du kit "Ethanol test®" de Boehringer (N° catalogue 176290). Les souches utilisées ont été LVA1531 (*ggs1−1*)et LVA4012=LVA1531 + pFPS1 (YEplac195/URA3 + *FPS1* sur un fragment SalI-HindIII), le gène *FPS1* étant décrit et séquencé dans la référence suivante : Van Aelst L, Hohmann S, Zimmermann K, Jans AWH & Thevelein JM 1991, EMBO J., 10, pp. 2095-2104.

Le mutant *ggs1−1*a une production d'éthanol réduite parce que le début exagérément actif de la glycolyse perturbe l'équilibre délicat entre la première et la seconde moitié de la chaîne glycolytique, conduisant à une diminution rapide du phosphate et de l'ATP.

La présence du gène supresseur *FPS1* augmente à nouveau la vitesse de la glycolyse. Apparemment, le facteur limitant qui semble être la disponibilité du phosphate libre nécessaire à la réaction catalysée par la glyceraldehyde-3-phosphate déshydrogénase, est au moins partiellement levée par le supresseur *FPS1*, dont on pense qu'il code pour une protéine de transport des phosphates (figure 3).

Une augmentation supplémentaire de la vitesse de la glycolyse peut être obtenue par une surexpression plus grande du gène *FPS1* et/ou la surexpression de gènes disposés en aval de la glycolyse, par exemple, après l'étape fructose 1,6-bisphosphate. De façon similaire, la même augmentation de la vitesse de fermentation peut être obtenue en introduisant une ou plusieurs mutations ponctuelles spécifiques dans le gène *GGS1*, son promoteur (dont on a vu que le changement pouvait induire un doublement de la Vmax caractérisant la cinétique d'entrée du glucose dans les cellules), sa séquence 3'-flanquante ou dans un ou plusieurs autres composants du complexe général senseur de glucose. Ceci aura un effet plus limité sur le transport du glucose et provoquera en lui-même (et également en combinaison avec les autres manipulations décrites ci-dessus) une augmentation de la vitesse de fermentation.

L'effet du clonage dans le mutant ggs1-1 des gènes FPS1 (souche LVA4012 : ggs1-1 + pFPS1) et GGS1 (souche LVA4020 : ggs1-1 + pGGS1) sur les activités invertase et alpha-glucosidase a également été étudiée. Les cellules de levure ont été cultivées sur le milieu glucose YPD et les activités enzymatiques mesurées sur des cellules en phase exponentielle de croissance (en présence de glucose) et des cellules déréprimées (milieu épuisé en glucose). Des méthodes standard de détermination des activités spécifiques enzymatiques ont été employées pour l'invertase (Golstein A & Lampen JO 1975, Methods Enzymol., 42C, pp. 504-511; Celenza JL & Carlson M 1984, Mol. Cell. Biol., 4 , pp. 49-53) excepté que la mesure a été faite à pH 5,1 et à 37°C; de même que pour l'activité alpha-glucosidase (Zimmerman FK et al 1977, Mol. Gen. Genet., 151, pp. 95-103). Les résultats obtenus (tableau 3) montrent que GGS1 restaure l'effet de répression par le glucose sur les deux systèmes enzymatiques, tandis que FPS1 ne le restaure pas ou seulement partiellement. De manière surprenante, il a été constaté que le clonage de FPS1 dans un mutant ggs1 entraine une augmentation considérable de l'activité invertase et une augmentation importante de l'activité alpha-glucosidase par rapport aux deux autres souches testées.

TABLEAU 3

| Effet du clonage de FPS1 ou GGS1 dans un mutant ggs1-1 sur les activités spécifiques invertase et alpha-glucosidase exprimées en nmol/min/mg de protéines | | | | |
|---|---|---|---|---|
| | Activité spécifique invertase | | Activité spécifique alpha-glucosidase | |
| | A | B | A | B |
| type sauvage (SP1) | 107 | 1676 | 16,4 | 45,2 |
| ggs1-1 + pFPS1 (LVA4012) | 30333 | 31995 | 60,8 | 83,6 |
| ggs1-1 + pGGS1 (LVA4020) | 329 | 1989 | 18,2 | 77,8 |
| A) Cellules en phase exponentielle cultivées sur glucose<br>B) Cellules déréprimées (glucose épuisé) | | | | |

**Revendications**

1. Souche de levure transformée de manière à posséder une résistance au stress et/ou un métabolisme des sucres différent comme notamment un pouvoir fermentatif amélioré, caractérisé par le fait d'une part que cette souche possède par rapport à une souche non transformée une modification d'au moins un de ses systèmes généraux senseurs de glucose composés au moins :
   - d'une protéine ayant une fonction de senseur général de glucose codée par le gène *GGS1* ou un gène similaire ;
   - d'une protéine transporteur membranaire (perméase) du glucose à faible affinité ;
   - d'une sucre kinase ;

   et par le fait d'autre part que la modification du ou des gènes codant pour ces protéines, et/ou de leur promoteur, et/ou de leur séquence 3'-flanquante, confère à la souche transformée des nouvelles propriétés pour sa production et/ou son utilisation comme levure à usage industriel.

2. Souche de levure transformée selon la revendication 1, caractérisée par le fait qu'elle possède par rapport à la souche non transformée une modification d'au moins un de ses gènes *GGS1* ou de ses allèles ou des gènes ayant, au moins partiellement, la même fonction que ce gène *GGS1*, ou de leur promoteur ou leur séquence 3'-flanquante.

3. Souche de levure transformée selon les revendications 1 ou 2 caractérisée en ce qu'un ou plusieurs gènes *GGS1* ou ses allèles ont été mis sous promoteur constitutif rendant son expression au moins partiellement indépendante de toute régulation par le glucose et/ou l'azote présents dans le milieu ou sous tout autre promoteur, comme les promoteurs forts, inductibles ou conditionnels.

4. Souche de levure transformée selon la revendication 3, caractérisée en ce qu'un ou plusieurs gènes *GGS1* ou ses allèles ont été mis sous promoteur *PDC1* ou *PGK*.

5. Souche de levure transformée selon l'une quelconque des revendications précédentes, caractérisée en ce qu'au moins un gène *GGS1* et/ou son promoteur et/ou sa séquence 3'-flanquante, est muté de manière à ce que sa transformation entraîne spécifiquement une activation de la synthèse du tréhalose et/ou une inactivation au moins partielle de la mobilisation du tréhalose.

6. Souche de levure transformée selon l'une quelconque des revendications précédentes caractérisée en ce que la mutation du ou des gènes *GGS1* est identique à celle de l'allèle *ggs1−3*.

**7.** Souche de levure transformée selon l'une quelconque des revendication 1 à 5 caractérisée en ce que la mutation d'au moins un des gènes *GGS1* ou ses allèles ou ses équivalents porte sur ses sites de phosphorylation.

**8.** Souche de levure transformée selon l'une des revendications précédentes, caractérisée par le fait qu'elle est plus résistante au stress que la souche non transformée, soit qu'elle acquiert dans les mêmes conditions de culture une plus grande résistance au stress, soit que la perte de sa résistance au stress est retardée, soit qu'elle cumule ces deux propriétés.

**9.** Souche de levure transformée selon la revendication 8, caractérisée par le fait que sa résistance au stress est indépendante ou moins dépendante du glucose et/ou de l'azote présents dans son milieu de culture ou de fermentation.

**10.** Souche de levure transformée selon la revendication 1 ou 2, caractérisée en ce qu'elle permet une entrée plus rapide du glucose mais à une vitesse telle que la glycolyse soit assurée.

**11.** Souche de levure transformée selon la revendication 10, caractérisée en ce que les gènes de la glycolyse ou les gènes codant pour des transporteurs de phosphate sont surexprimés ou renforcés

**12.** Souche de levure transformée selon l'une des revendications 10 ou 11, caractérisée en ce que le gène *FPS1* est surexprimé ou renforcé.

**13.** Souche de levure transformée selon l'une quelconque des revendications 10 à 12, caractérisée en ce que la souche de levure transformée présente par rapport à la souche non transformées une augmentation de son activité fermentative déterminée par la vitesse de transformation des sucres présents en éthanol et en gaz carbonique et/ou un taux de croissance amélioré.

**14.** Souche de levure transformée selon la revendication 1 ou 2, caractérisée en ce que la transformation du système général senseur de glucose est dirigée de manière à obtenir un changement spécifique des signaux de régulation induits par la présence de glucose et/ou les signaux de régulation commandant le métabolisme des sucres.

**15.** Souche de levure transformée selon la revendication 3, caractérisée en ce que le gène *GGS1* ou un de ses allèles est placé sous le contrôle d'un promoteur constitutif de manière à ce qu'il soit un marqueur de sélection dominante positif associé à tout autre transformation par ADN recombinant d'une souche de levure industrielle.

**16.** Souche de levure selon l'une quelconque des revendications précédentes, caractérisée en ce que les transformations désirées de la souche de départ sont réalisées par une autre méthode que la recombinaison génétique par ADN transformant.

**17.** Souche de levure selon la revendication 16, caractérisée en ce que la souche de levure ayant les modifications recherchées de son génome, est susceptible d'être obtenue par le procédé consistant à mutagéniser par des techniques classiques les souches de levures industrielles de départ ou leurs descendants haploïdes, à isoler par PCR dans les souches mutées les allèles *GGS1* sous leur propre promoteur, à introduire lesdits allèles dans une souche *ggs1*Δ, à sélectionner les transformants qui en résultent selon les critères recherchés, et à croiser, par croisements successifs, le mutant haploïde ayant l'allèle de *GGS1* portant les propriétés désirées avec des haploïdes de la souche industrielle de départ, jusqu'à obtention d'une nouvelle souche industrielle ayant gardé l'ensemble de ses propriétés intéressantes de départ et possèdant en plus les nouvelles propriétés conférées par l'allèle de *GGS1* ainsi sélectionné.

**18.** Souche de levure selon l'une quelconque des revendications précédentes, caractérisées en ce que la souche de levure appartient avantageusement à l'espèce Saccharomyces cerevisiae.

**19.** Souche de levure selon l'une quelconque des revendications précédentes, caractérisée en ce qu'elle est de préférence une souche de levure de panification.

**20.** Souche de levure de panification selon la revendication 19, caractérisée en ce qu'elle est plus résistante au stress, c'est-à-dire qu'elle possède l'une au moins des caractéristiques suivantes par rapport à la souche non transformée cultivée dans les mêmes conditions :
- meilleure résistance au séchage;
- meilleure résistance aux chocs osmotiques, notamment dans les pâtes sucrées;
- meilleure résistance à la congélation;
- meilleure survie dans les pâtes boulangères congelées ou de préférence plusieurs de ces caractéristiques.

**21.** Souche de levure de panification selon la revendication 20, caractérisée en ce qu'elle possède par rapport à la souche non transformée cultivée dans les mêmes conditions, au moins l'une des propriétés suivantes :
- une teneur plus importante en tréhalose ;
- un retard dans la mobilisation du tréhalose.

**22.** Utilisation d'une souche de levure selon les revendications précédentes, en panification ou production d'alcool ou production de boissons, ou production de protéines hétérologues, ou production de biomasses de levures.

FIG. 1

```
-793 TTTTTTAAACGTATATAGATGTCTACATGTGTGTTTTTGT -757
-756 TTTTTTACGTACGTATACCCACCTATATATGCATAATCCGTAATTGAAAAAAAAAAAAGAAAA -694
-693 AGATCAAGGAACACATCACCCTGGGCACATCAAGCGTGAGGAATGCCGTCCAACTGGTGGAGA -631
-630 CGCTTGATTTGCTCTTTTTGTTCTGGGTCCAACCCGGTCTCGAAGAACATCAGCACCACGCCC -568
-567 GCAACGACAAAGAACATTGCAATACACTTGCATATGTGAGCATAGTCGAGCGGTCCGTTCTGT -505
-504 GGTTGATGCTGTTGTTCTTTCTTCTGTTTGTCAGGGGTGATAGCCATATCTTCGTGCTCTTGT -442
-441 TGCGATTGTTCTGTTCCATCTGCACCAGAACAAAGAACAAAAGAACAAGGAACAAAGTCCAAG -379
-378 CACGTCAGCGCTGTTTATAAGGGGATTGACGAGGGATCGGGCCTAGAGTGCCAGCGCGCCAGG -316
-315 GAGAGGGAGCCCCCTGGGCCCTCATCCGCAGGCTGATAGGGGTCACCCCGCTGGGCAGGTCAG -253
-252 GGCAGGGGCTCTCAGGGGGGCGCCATGGACAAACTGCACTGAGGTTCTAAGACACATGTATTA -190
-189 TTGTGAGTATGTATATATAGAGAGAGATTAAGGCGTACAGCCGGGTGGTAGAGATTGATTAAC -127
-126 TTGGTAGTCTTATCTTGTCAATTGAGTTTCTGTCAGTTTCTTCTTGAACAAGCACGCAGCTAA  -64
 -63 GTAAGCAACAAAGCAGGCTAACAAACTAGGTACTCACATACAGACTTATTAAGACATAGAACT   -1

   1 Met Thr Thr Asp Asn Ala Lys Ala Gln Leu Thr Ser Ser Ser Gly Gly   16
   1 ATG ACT ACG GAT AAC GCT AAG GCG CAA CTG ACC TCG TCT TCA GGG GGT   48

  17 Asn Ile Ile Val Val Ser Asn Arg Leu Pro Val Thr Ile Thr Lys Asn   32
  49 AAC ATT ATT GTG GTG TCC AAC AGG CTT CCC GTG ACA ATC ACT AAA AAC   96

  33 Ser Ser Thr Gly Gln Tyr Glu Tyr Ala Met Ser Ser Gly Gly Leu Val   48
  97 AGC AGT ACG GGA CAG TAC GAG TAC GCA ATG TCG TCC GGA GGG CTG GTC  144

  49 Thr Ala Leu Glu Gly Leu Lys Lys Thr Tyr Thr Phe Lys Trp Phe Gly   64
 145 ACG GCG TTG GAA GGG TTG AAG AAG ACG TAC ACT TTC AAG TGG TTC GGA  192

  65 Trp Pro Gly Leu Glu Ile Pro Asp Asp Glu Lys Asp Gln Val Arg Lys   80
 193 TGG CCT GGG CTA GAG ATT CCT GAC GAT GAG AAG GAT CAG GTG AGG AAG  240

  81 Asp Leu Leu Glu Lys Phe Asn Ala Val Pro Ile Phe Leu Ser Asp Glu   96
 241 GAC TTG CTG GAA AAG TTT AAT GCC GTA CCC ATC TTC CTG AGC GAT GAA  288

  97 Ile Ala Asp Leu His Tyr Asn Gly Phe Ser Asn Ser Ile Leu Trp Pro  112
 289 ATC GCA GAC TTA CAC TAC AAC GGG TTC AGT AAT TCT ATT CTA TGG CCG  336

 113 Leu Phe His Tyr His Pro Gly Glu Ile Asn Phe Asp Glu Asn Ala Trp  128
 337 TTA TTC CAT TAC CAT CCT GGT GAG ATC AAT TTC GAC GAG AAT GCG TGG  384

 129 Leu Ala Tyr Asn Glu Ala Asn Gln Thr Phe Thr Asn Glu Ile Ala Lys  144
 385 TTG GCA TAC AAC GAG GCA AAC CAG ACG TTC ACC AAC GAG ATT GCT AAG  432

 145 Thr Met Asn His Asn Asp Leu Ile Trp Val His Asp Tyr His Leu Met  160
 433 ACT ATG AAC CAT AAC GAT TTA ATC TGG GTG CAT GAT TAC CAT TTG ATG  480

 161 Leu Val Pro Glu Met Leu Arg Val Lys Ile His Glu Lys Gln Leu Gln  176
 481 TTG GTT CCG GAA ATG TTG AGA GTC AAG ATT CAC GAG AAG CAA CTG CAA  528

 177 Asn Val Lys Val Gly Trp Phe Leu His Thr Pro Phe Pro Ser Ser Glu  192
 529 AAC GTT AAG GTC GGG TGG TTC CTG CAC ACA CCA TTC CCT TCG AGT GAA  576

 193 Ile Tyr Arg Ile Leu Pro Val Arg Gln Glu Ile Leu Lys Gly Val Leu  208
 577 ATT TAC AGA ATC TTA CCT GTC AGA CAA GAG ATT TTG AAG GGT GTT TTG  624

 209 Ser Cys Asp Leu Val Gly Phe His Thr Tyr Asp Tyr Ala Arg His Phe  224
 625 AGT TGT GAT TTA GTC GGG TTC CAC ACA TAC GAT TAT GCA AGA CAT TTC  672

 225 Leu Ser Ser Val Gln Arg Val Leu Asn Val Asn Thr Leu Pro Asn Gly  240
 673 TTG TCT TCC GTG CAA AGA GTG CTT AAC GTG AAC ACA TTG CCT AAT GGG  720

 241 Val Glu Tyr Gln Gly Arg Phe Val Asn Val Gly Ala Phe Pro Ile Gly  256
 721 GTG GAA TAC CAG GGC AGA TTC GTT AAC GTA GGG GCC TTC CCT ATC GGT  768

 257 Ile Asp Val Asp Lys Phe Thr Asp Gly Leu Lys Lys Glu Ser Val Gln  272
 769 ATC GAC GTG GAC AAG TTC ACC GAT GGG TTG AAA AAG GAA TCC GTA CAA  816

 273 Lys Arg Ile Gln Gln Leu Lys Glu Thr Phe Lys Gly Cys Lys Ile Ile  288
 817 AAG AGA ATC CAA CAA TTG AAG GAA ACT TTC AAG GGC TGC AAG ATC ATA  864

 289 Val Gly Val Asp Arg Leu Asp Tyr Ile Lys Gly Val Pro Gln Lys Leu  304
 865 GTT GGT GTC GAC AGG CTG GAT TAC ATC AAA GGT GTG CCT CAG AAG TTG  912

 305 His Ala Met Glu Val Phe Leu Asn Glu His Pro Glu Trp Arg Gly Lys  320
 913 CAC GCC ATG GAA GTG TTT CTG AAC GAG CAT CCA GAA TGG AGG GGC AAG  960

 321 Val Val Leu Val Gln Val Ala Val Pro Ser Arg Gly Asp Val Glu Glu  336
 961 GTT GTT CTG GTA CAG GTT GCA GTG CCA AGT CGT GGA GAT GTG GAA GAG 1008

 337 Tyr Gln Tyr Leu Arg Ser Val Val Asn Glu Leu Val Gly Arg Ile Asn  352
1009 TAC CAA TAT TTA AGA TCT GTG GTC AAT GAG TTG GTC GGT AGA ATC AAC 1056

 353 Gly Gln Phe Gly Thr Val Glu Phe Val Pro Ile His Phe Met His Lys  368
1057 GGT CAG TTC GGT ACT GTG GAA TTC GTC CCC ATC CAT TTC ATG CAC AAG 1104

 369 Ser Ile Pro Phe Glu Glu Leu Ile Ser Leu Tyr Ala Val Ser Asp Val  384
1105 TCT ATA CCA TTT GAA GAG CTG ATT TCG TTA TAT GCT GTG AGC GAT GTT 1152

 385 Cys Leu Val Ser Ser Thr Arg Asp Gly Met Asn Leu Val Ser Tyr Glu  400
1153 TGT TTG GTC TCG TCC ACC CGT GAT GGT ATG AAC TTG GTT TCC TAC GAA 1200

 401 Tyr Ile Ala Cys Gln Glu Glu Lys Lys Gly Ser Leu Ile Leu Ser Glu  416
1201 TAT ATT GCT TGC CAA GAA GAA AAG AAA GGT TCC TTA ATC CTG AGT GAG 1248

 417 Phe Thr Gly Ala Ala Gln Ser Leu Asn Gly Ala Ile Ile Val Asn Pro  432
1249 TTC ACA GGT GCC GCA CAA TCC TTG AAT GGT GCT ATT ATT GTA AAT CCT 1296

 433 Trp Asn Thr Asp Asp Leu Ser Asp Ala Ile Asn Glu Ala Leu Thr Leu  448
1297 TGG AAC ACC GAT GAT CTT TCT GAT GCC ATC AAC GAG GCC TTG ACT TTG 1344

 449 Pro Asp Val Lys Lys Glu Val Asn Trp Glu Lys Leu Tyr Lys Tyr Ile  464
1345 CCC GAT GTA AAG AAA GAA GTT AAC TGG GAA AAA CTT TAC AAA TAC ATC 1392

 465 Ser Lys Tyr Thr Ser Ala Phe Trp Gly Glu Asn Phe Val His Glu Leu  480
1393 TCT AAA TAC ACT TCT GCC TTC TGG GGT GAA AAT TTC GTC CAT GAA TTA 1440

 481 Tyr Ser Thr Ser Ser Ser Ser Thr Ser Ser Ser Ala Thr Lys Asn ***  496
1441 TAC AGT ACA TCA TCA AGC TCA ACA AGC TCC TCT GCC ACC AAA AAC TGA 1488

1489 TGAACCCGATGCAAATGAGACGATCGTCTATTCTGGTCCGGTTTTCTCTGCCCTCTCTTCTAT 1552
1553 TCACTTTTTTTATACTTTATATAAAATTATATAAATGACATAACTGAACGCCACACGTCCTCT 1615
1616 CCTATTCGTTAACGCCTGTCTCTAGCGCTGTTACTGAAGCTGCGCAAGTAGTTTTTTCACCGT 1678
1679 ATAGGCCCTCTTTTTCTCTCTCTTTCTTTCTCTCCCGCGCTGATCTCTTCTTCGAAACATCAT 1741
1742 GAATAAAAAGAAAAAGGAAATCAAGAAAAAAAAAGCCATAATTTATCCCACATTTT 1796
```

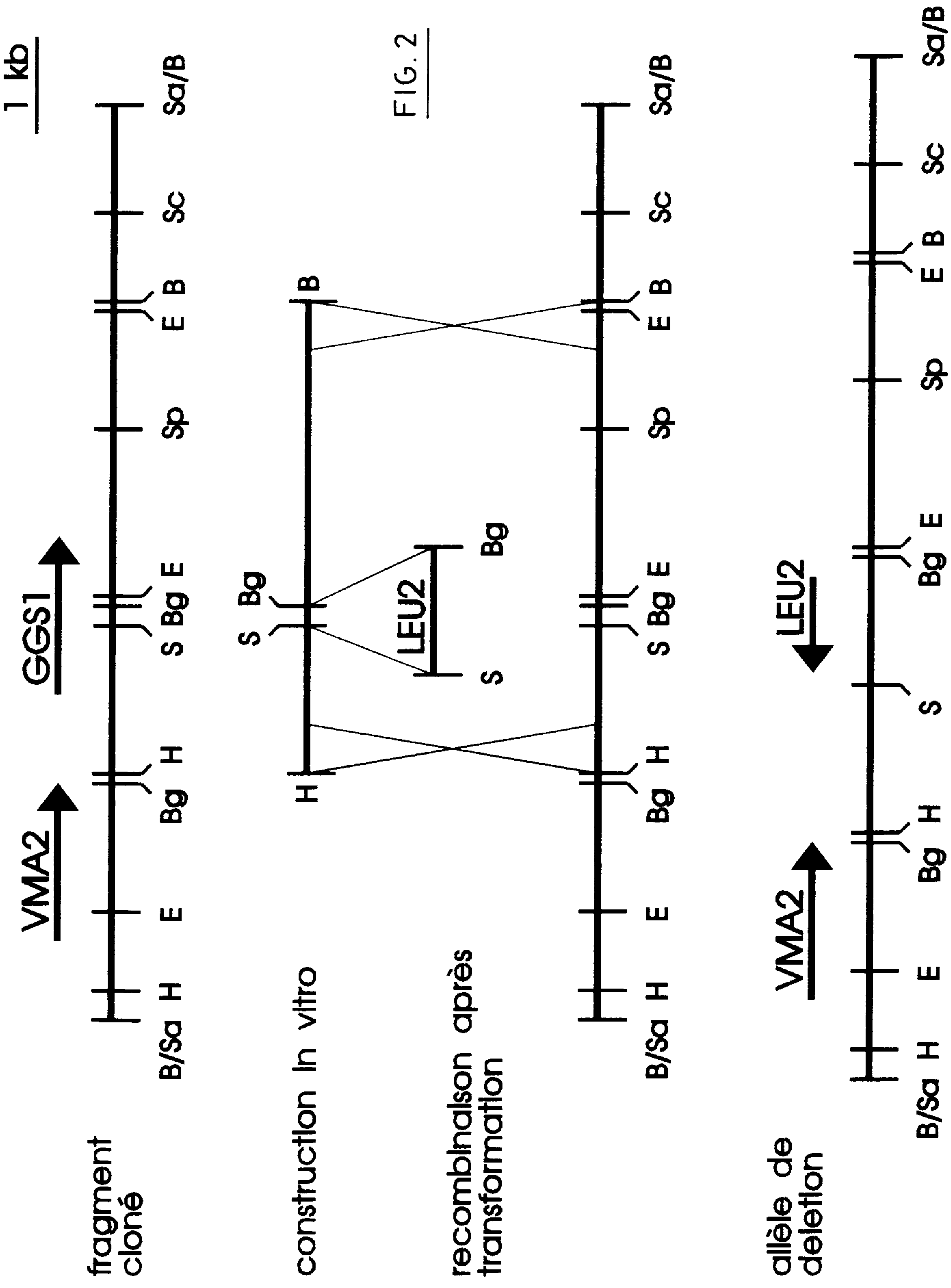
1 kb
fragment cloné
VMA2
GGS1
B/Sa H E Bg H S Bg E Sp E B Sc Sa/B
construction in vitro
H S Bg B
LEU2
S Bg
recombinaison après transformation
B/Sa H E Bg H S Bg E Sp E B Sc Sa/B
allèle de deletion
VMA2
LEU2
B/Sa H E Bg H S Bg E Sp E B Sc Sa/B

FIG. 2

FIG. 3

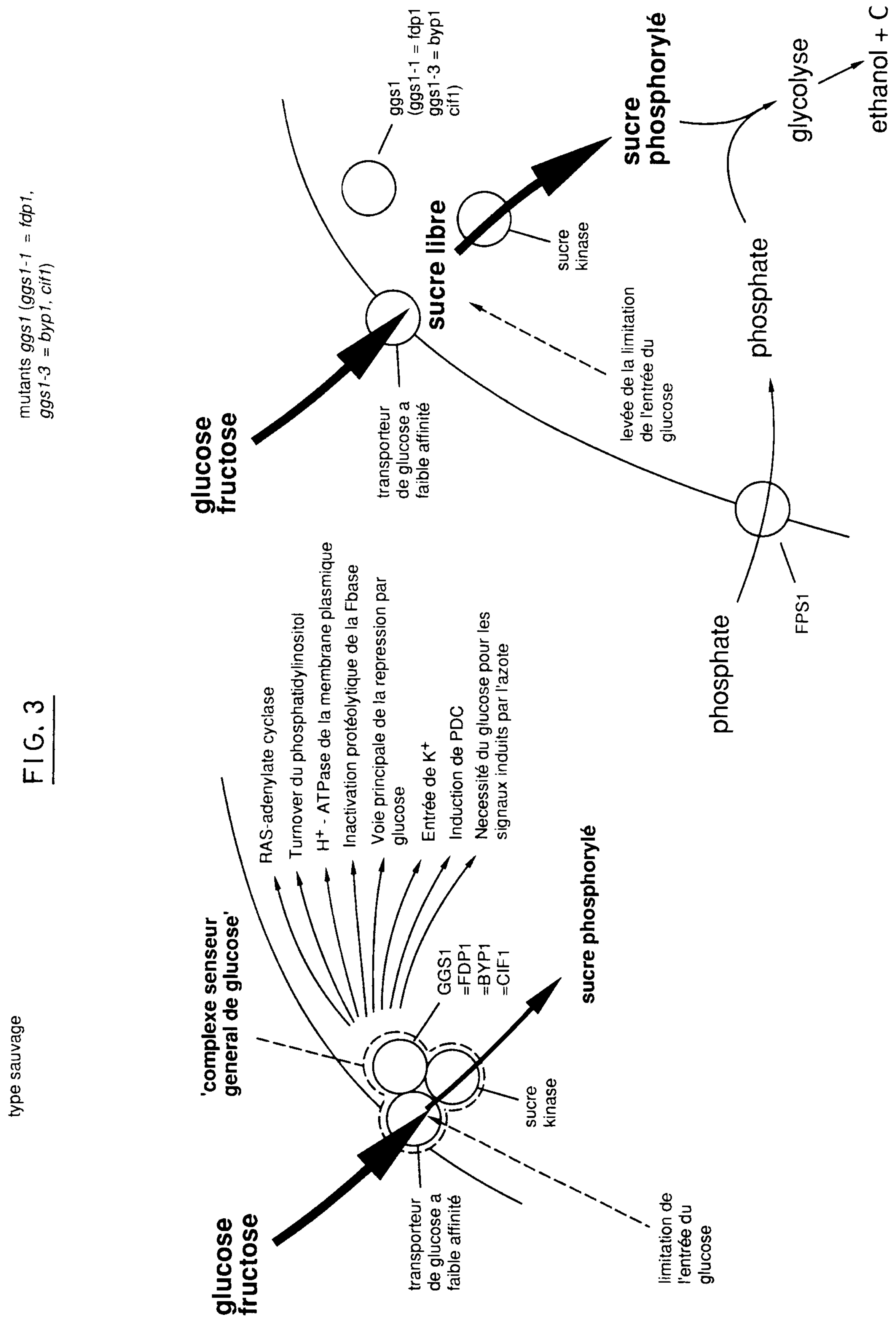
type sauvage
'complexe senseur general de glucose'
glucose fructose
transporteur de glucose a faible affinité
limitation de l'entrée du glucose
sucre kinase
GGS1 =FDP1 =BYP1 =CIF1
sucre phosphorylé
RAS-adenylate cyclase
Turnover du phosphatidylinositol
$H^+$ - ATPase de la membrane plasmique
Inactivation protéolytique de la Fbase
Voie principale de la repression par glucose
Entrée de $K^+$
Induction de PDC
Necessité du glucose pour les signaux induits par l'azote
mutants ggs1 (ggs1-1 = fdp1, ggs1-3 = byp1, cif1)
glucose fructose
transporteur de glucose a faible affinité
sucre libre
levée de la limitation de l'entrée du glucose
ggs1 (ggs1-1 = fdp1 ggs1-3 = byp1 cif1)
sucre kinase
sucre phosphorylé
phosphate
phosphate
FPS1
glycolyse
ethanol + C

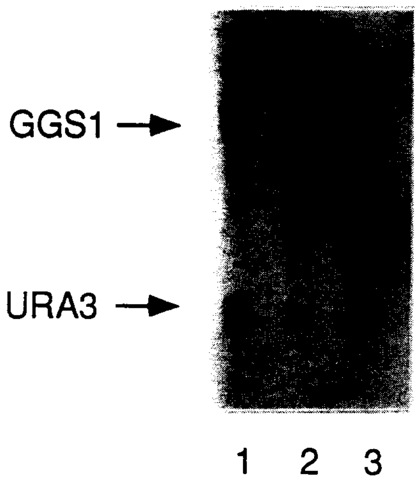


FIG. 4

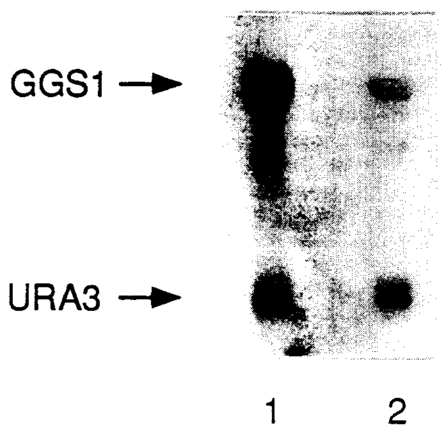


FIG. 5

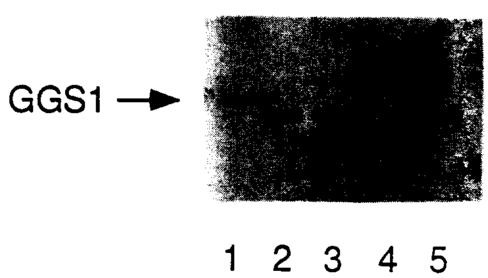


FIG. 6

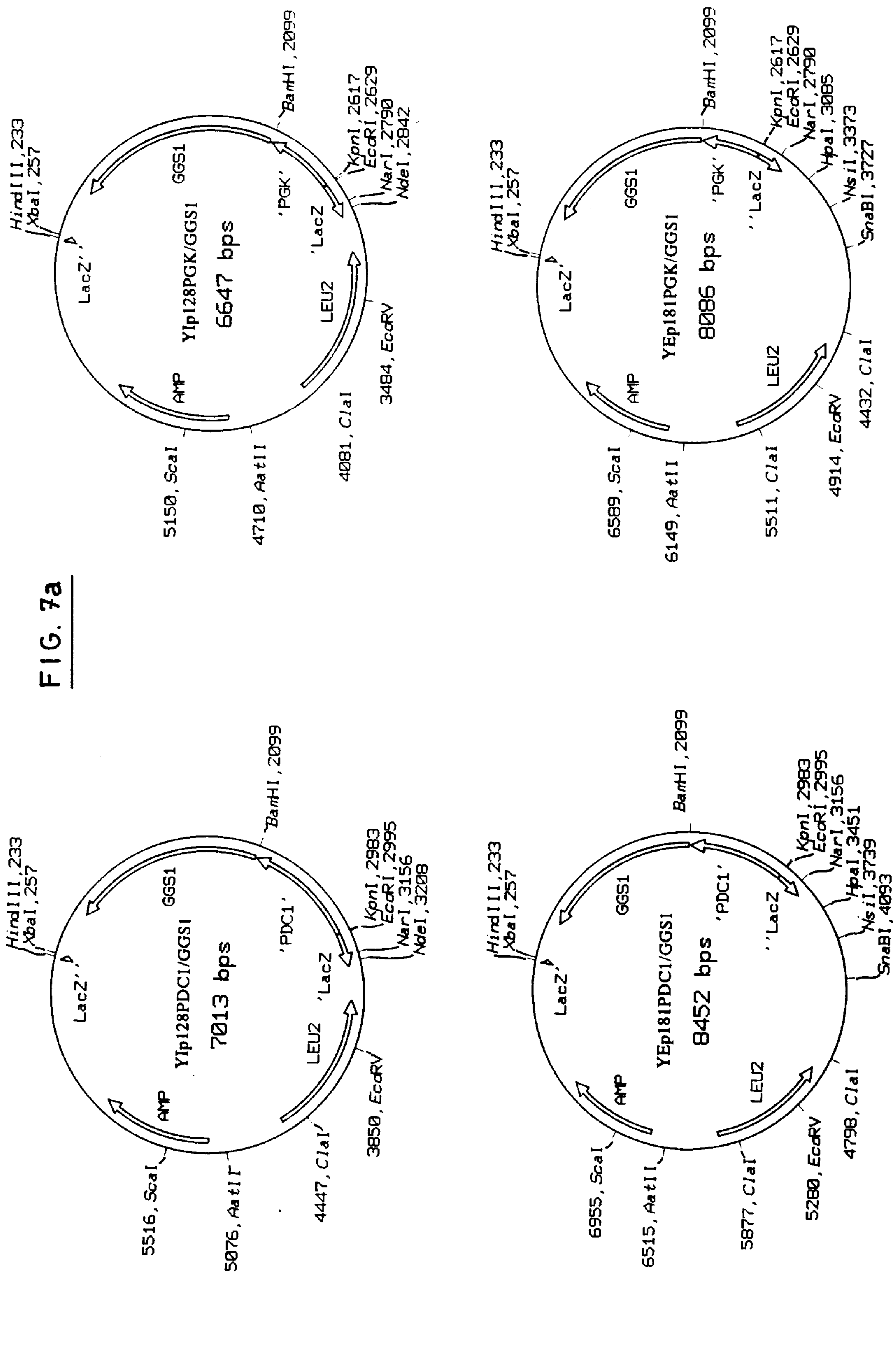
YIp128PGK/GGS1
6647 bps
HindIII,233
XbaI,257
BamHI,2099
KpnI,2617
EcoRI,2629
NarI,2790
NdeI,2842
3484,EcoRV
4081,ClaI
4710,AatII
5150,ScaI
LacZ'
GGS1
'PGK'
'LacZ
LEU2
AMP
YEp181PGK/GGS1
8086 bps
HindIII,233
XbaI,257
BamHI,2099
KpnI,2617
EcoRI,2629
NarI,2790
HpaI,3085
NsiI,3373
SnaBI,3727
4432,ClaI
4914,EcoRV
5511,ClaI
6149,AatII
6589,ScaI
''LacZ
YIp128PDC1/GGS1
7013 bps
HindIII,233
XbaI,257
BamHI,2099
KpnI,2983
EcoRI,2995
NarI,3156
NdeI,3208
3850,EcoRV
4447,ClaI
5076,AatII
5516,ScaI
'PDC1'
YEp181PDC1/GGS1
8452 bps
KpnI,2983
EcoRI,2995
NarI,3156
HpaI,3451
NsiI,3739
SnaBI,4093
4798,ClaI
5280,EcoRV
5877,ClaI
6515,AatII
6955,ScaI

FIG. 7a

FIG. 7b

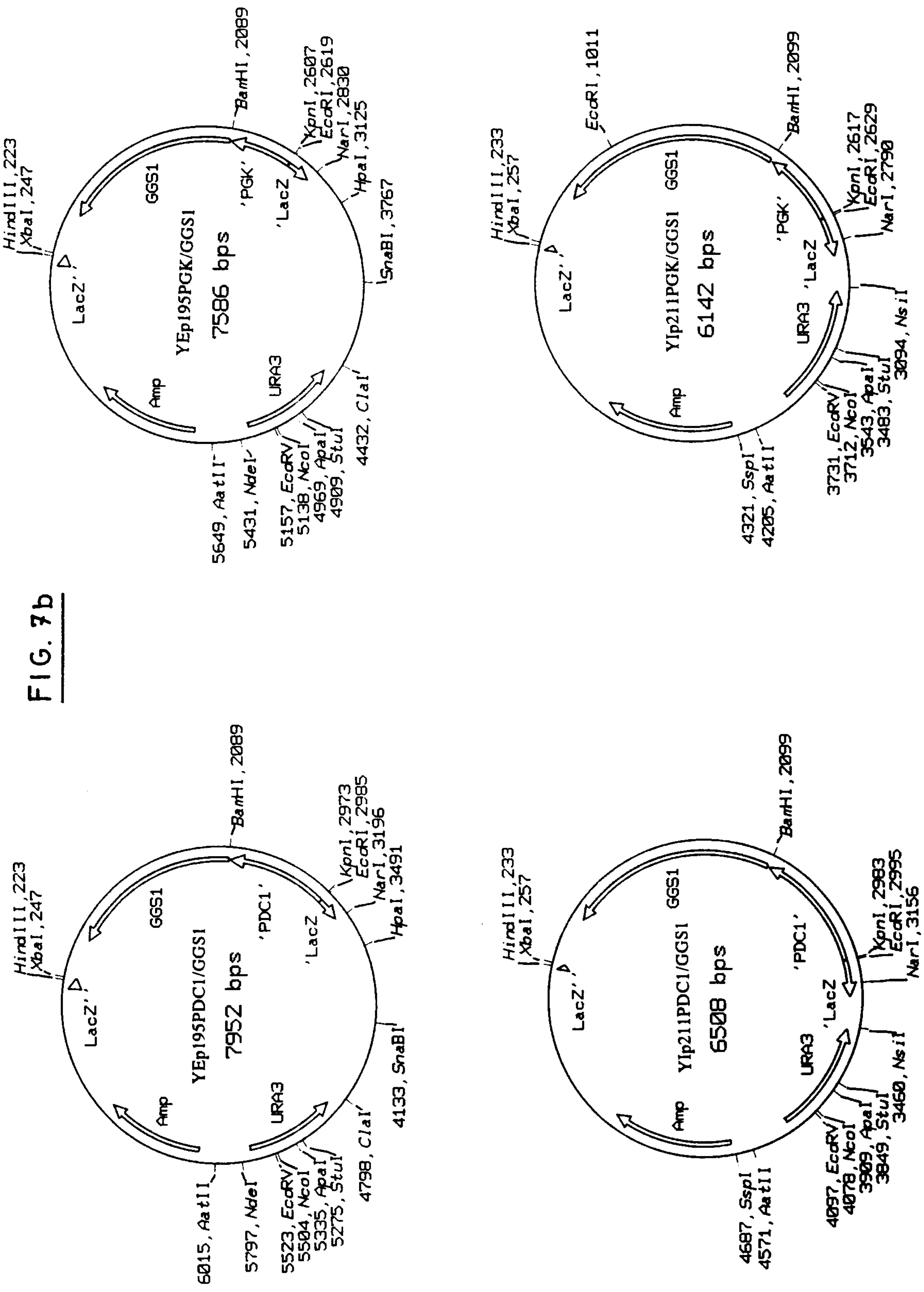
YEp195PGK/GGS1
7586 bps
HindIII,223
XbaI,247
BamHI,2089
KpnI,2607
EcoRI,2619
NarI,2830
HpaI,3125
SnaBI,3767
4432,ClaI
4909,StuI
4969,ApaI
5138,NcoI
5157,EcoRV
5431,NdeI
5649,AatII
GGS1
'PGK'
'LacZ
URA3
Amp
LacZ'
YIp211PGK/GGS1
6142 bps
HindIII,233
XbaI,257
EcoRI,1011
BamHI,2099
KpnI,2617
EcoRI,2629
NarI,2790
3094,NsiI
3483,StuI
3543,ApaI
3712,NcoI
3731,EcoRV
4205,AatII
4321,SspI
YEp195PDC1/GGS1
7952 bps
HindIII,223
XbaI,247
BamHI,2089
KpnI,2973
EcoRI,2985
NarI,3196
HpaI,3491
4133,SnaBI
4798,ClaI
5275,StuI
5335,ApaI
5504,NcoI
5523,EcoRV
5797,NdeI
6015,AatII
'PDC1'
YIp211PDC1/GGS1
6508 bps
HindIII,233
XbaI,257
BamHI,2099
KpnI,2983
EcoRI,2995
NarI,3156
3460,NsiI
3849,StuI
3909,ApaI
4078,NcoI
4097,EcoRV
4571,AatII
4687,SspI

FIG. 8

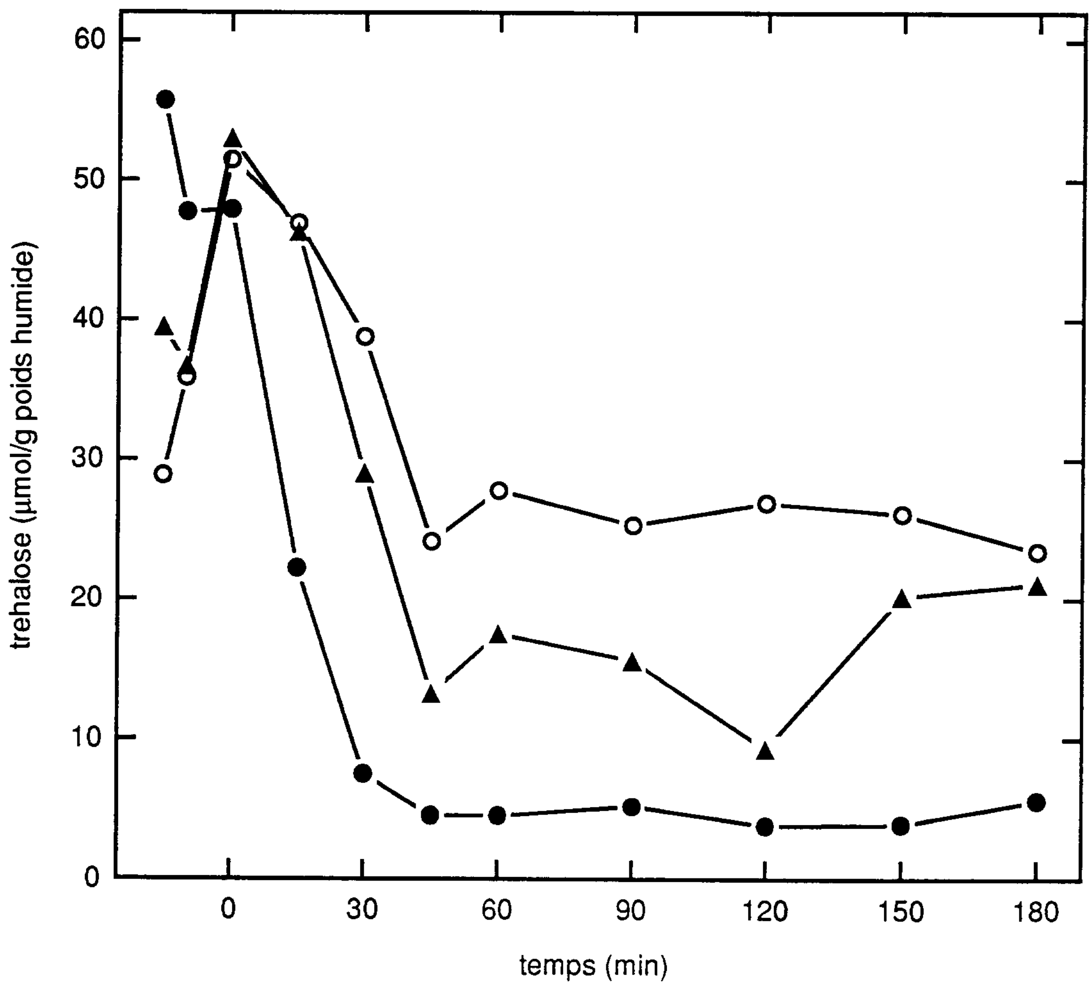
60
50
40
30
20
10
0
trehalose (µmol/g poids humide)
0
30
60
90
120
150
180
temps (min)

Office européen des brevets

# RAPPORT DE RECHERCHE EUROPEENNE

Numero de la demande

EP 92 87 0102
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | EP-A-0 306 107 (GIST-BROCADES N.V.)<br>* page 3, ligne 22 - ligne 49 *<br>--- | 1 | C12N15/81<br>C12N1/18<br>A21D8/04<br>C07K15/00<br>C12N9/12 |
| X | CHEMICAL ABSTRACTS, vol. 105, no. 17, Octobre 1986, Columbus, Ohio, US; abstract no. 151567c, H. KIMURA ET AL. 'ATP and ethanol production by genetically engineered yeasts.' page 569 ;colonne 2 ;<br>* abrégé *<br>& PATENT ABSTRACTS OF JAPAN<br>& JP-A-61 124 390 ( WAKO BIOK.K., NIKKA WHISKY DISTILLING CO., LTD. ) 12 Juin 1986<br>* abrégé *<br>--- | 1 | |
| A | EP-A-0 451 896 (GIST-BROCADES N.V.)<br>* le document en entier *<br>--- | 1-22 | |
| T | EUROPEAN JOURNAL OF BIOCHEMISTRY vol. 209, no. 3, 1 Novembre 1992, pages 951 - 959 W. BELL ET AL. 'Characterization of the 56-kDa subunit of yeast trehalose-6-phosphate synthase and cloning of its gene reveal its identity with the product of CIFI, a regulator of carbon catabolite inactivation.'<br>* le document en entier *<br>--- | 1-22 | DOMAINES TECHNIQUES RECHERCHES (Int. Cl.5 )<br>C12N |
| A | YEAST. vol. 5, 1989, J. WILEY & SONS, LTD. pages 537 - 540 A.D. PANEK & A.C. PANEK 'Trehalose enzymes as models for signal transduction in Saccharomyces.'<br>--- | | |
| A | WO-A-8 703 006 (GENETICS INSTITUTE, INV.)<br>----- | | |

Le présent rapport a été établi pour toutes les revendications

| Lieu de la recherche | Date d'achèvement de la recherche | Examinateur |
|---|---|---|
| LA HAYE | 05 MARS 1993 | BEVAN S.R. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

Office européen des brevets

# RAPPORT DE RECHERCHE EUROPEENNE

Numero de la demande

EP 92 87 0102
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| | | | |

DOMAINES TECHNIQUES RECHERCHES (Int. Cl.5 )

Le présent rapport a été établi pour toutes les revendications

| Lieu de la recherche | Date d'achèvement de la recherche | Examinateur |
|---|---|---|
| LA HAYE | 05 MARS 1993 | BEVAN S.R. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)